(19) Europäisches Patentamt
European Patent Office
Office européen des brevets

(11) Publication number: **0 038 117**
**A1**

(12) **EUROPEAN PATENT APPLICATION**

(21) Application number: **81300941.2**

(22) Date of filing: **06.03.81**

(51) Int. Cl.³: **C 07 D 207/16**, C 07 C 103/52, C 07 C 153/07, A 61 K 37/64, C 07 D 277/06

(30) Priority: **10.03.80 US 128953**
**10.06.80 US 158278**

(43) Date of publication of application: **21.10.81**
**Bulletin 81/42**

(84) Designated Contracting States: **BE CH DE FR GB IT LI LU NL SE**

(71) Applicant: **UNIVERSITY OF MIAMI, Coral Gables Florida 33124 (US)**

(72) Inventor: **Ryan, James Walter, 3420 Poinclana Avenue, Miami Florida 33133 (US)**
Inventor: **Chung, Alfred, 8781 Southwest 87th. Street, Miami Florida 33183 (US)**

(74) Representative: **De Minvielle-Devaux, Ian Benedict Peter, CARPMAELS & RANSFORD 43, Bloomsbury Square, London WC1A 2RA (GB)**

(54) **Methods for preparing anti-hypertensive agents.**

(57) A plurality of methods are disclosed for preparing compounds having the formula:

$$R - A - S - (CH_2)_n - \underset{\underset{R_1}{|}}{CH} - \overset{\overset{O}{\|}}{C} - R_2$$

wherein

R is hydrogen, formyl, acetyl, propanoyl, butanoyl, phenylacetyl, phenylpropanoyl, benzoyl, cyclopentanecarbonyl, tert-butyloxycarbonyl, cyclopentanecarbonyl-L-lysyl, pyro-L-glutamyl-L-lysyl, L-lysyl, L-arginyl or pyro-L-glutamyl;

A is phenylalanyl, alanyl, tryptophyl, tyrosyl, glycyl isoleucyl, leucyl, histidyl, or valyl, the $\alpha$-amino group thereof being in amide linkage with R;

$R_1$ is hydrogen or methyl,

$R_2$ is L-proline, L-3,4-dehydroproline, D,L-3,4-dehydroproline, L-3-hydroxyproline, L-4-hydroxyproline, L-thiazolidine-4-carboxylic acid, or L-5-oxo-proline, the imino group thereof being in imide linkage with the adjacent $-\overset{\overset{O}{\|}}{C}-$; and,

n is 0 or 1, such that when n is 0, $R_1$ is methyl, and where n=1, and $R_1$ is methyl the $-CH_2-\underset{\underset{CH_3}{|}}{CH}-$ moiety is in the D-configuration. These compounds are inhibitors of angiotensin converting enzyme.

# METHODS FOR PREPARING ANTI-HYPERTENSIVE AGENTS

## BACKGROUND OF THE INVENTION

Angiotensin converting enzyme (peptidyldipeptide hydrolase, hereinafter referred to as ACE) occupies a central role in the physiology of hypertension. The enzyme is capable of converting the decapeptide angiotensin I, having the sequence

AspArgValTyrIleHisProPheHisLeu

to an octapeptide, angiotensin II, by catalyzing the hydrolysis of angiotensin I's penultimate peptide bond, and thereby effecting the removal of the carboxyl - terminal HisLeu.

The symbols for various chemical entities are explained in the following table:

ACE = Angiotensin converting enzyme
Ala = L-alanine
Arg = L-arginine
Asp = L-aspartic acid
Boc = t-butyloxycarbonyl
Bpoc = 2-(4-biphenyl)-2-propyloxycarbonyl
Bz = benzoyl
Cbo = benzyloxycarbonyl
Ddz = N-α,α-dimethyl-3,5-dimethyloxycarbonyl
Gly = glycine
His = L-histidine
Ile = L-isoleucine
Leu = L-leucine
Phe = L-phenylalanine
Ppoc = 2-phenylisopropyloxycarbonyl
Pro = L-proline
ΔPro = L-3,4-dehydroproline
Ser = L-serine
Trp = L-tryptophan
Tyr = L-tyrosine
Val = L-valine

Angiotensin II is a powerful pressor (blood pressure elevating) agent. In addition to its direct pressor effect, angiotensin II stimulates release of aldosterone which tends to elevate blood pressure by causing retention of extracellular salt and fluids. Angiotensin II is found in

measurable amount in the blood of normal humans. However, it is found at elevated concentrations in the blood of patients with renal hypertension.

The level of ACE activity is ordinarily in excess, in both normal and hypertensive humans, of the amount needed to maintain observed levels of angiotensin II. However, it has been found that significant blood pressure lowering is achieved in hypertensive patients by treatment with ACE inhibitors. [Gavras, I., et al., New Engl. J. Med. 291, 817 (1974)].

The role of ACE in the pathogenesis of hypertension has prompted a search for inhibitors of the enzyme that could act as antihypertensive drugs. See for example U.S. Patents 3,891,616, 3,947,575, 4,052,511 and 4,053,651. The reactivity of ACE varies markedly depending on the substrate. Many peptides which are called inhibitors of the enzymatic conversion of angiotensin I to angiotensin II are in fact substrates having a lower Michaelis constant (Km) than angiotensin I. Such peptides are more properly termed competitive substrates.

Numerous synthetic peptide derivatives have been shown to be ACE inhibitors by Ondetti, et al. in U.S. Patent 3,832,337 issued August 27, 1974. A highly effective inhibitor, with high biological activity when orally administered, is D-3-mercapto-2-methylpropanoyl-L-proline, designated SQ14225, disclosed in U.S. Patent 4,046,889 to Ondetti et al., issued September 6, 1977, and in scientific articles by Cushman, D.W. et al., Biochemistry 16, 5484 (1977), and by Ondetti, M. et al., Science 196, 441 (1977). The inhibitor SQ14225 reportedly has an $I_{50}$ value of 2.3 x $10^{-8}$M. The $I_{50}$ value reported by Cushman, et al., supra is the concentration of inhibitor required to produce 50% inhibition of the enzyme under a standard assay system containing substrate at a level substantially above $K_m$. It will be understood that $I_{50}$ values are directly comparable when all potential factors affecting the reaction are kept constant. These factors include the source of enzyme, its purity, the substrate used and its concentration, and the composition of

the assay buffer. All $I_{50}$ data reported herein have been performed with the same assay system and same enzyme (human urinary ACE) and with an approximately 1/2 $K_m$ level of substrate and are therefore internally consistent.

The mode of action of SQ14225 has been based upon a model of the active site of ACE developed by analogy with the better known related enzyme, carboxypeptidase A. The active site was postulated to have a cationic site for binding the carboxyl end group of the substrate and a pocket or cleft capable of binding the side chain of the C-terminal amino acid and providing especially tight binding for the heterocyclic ring of a terminal proline residue. A similar pocket for the penultimate amino acid residue was postulated, and the published data suggested a rather stringent steric requirement, since the D-form of the inhibitor was substantially more potent than its stereoisomer or the 3-methyl and unsubstituted analogs. The sulfhydryl group on the inhibitor, postulated to be bound at the active site near the catalytic center, was believed to play a central role in inactivation of the enzyme by combining with the zinc moiety known to be essential for catalytic activity. Substituents on the sulfhydryl, such as a methyl group, and an S-acetyl derivative, substantially reduced potency of the inhibitor. See Cushman, D.W., et al., Biochemistry, supra.

In vitro study of the mechanism by which SQ14225 and its analogs act to inhibit ACE has been somewhat hampered by the instability of these molecules under ambient conditions. For example, it has been observed that a fresh aqueous solution of concentration, e.g., 1 mg per ml of SQ14225 at a pH of about 8 becomes substantially less active upon standing for as little as 30 minutes, and that activity continues to decrease as the solution stands for longer periods. It is believed that this loss in activity is mainly the result of dimerization of SQ14225 occurring at the sulfhydryl end groups, whereby a disulfide is formed which is largely inactive as an inhibitor. Since the free sulfhydryl group is highly reactive and may be readily oxidized to polar acidic moieties such as sulfone and sulfoxide groups, it may also be

that the observed _in vitro_ loss of activity of aqueous solutions of SQ14225 on standing is in some part a consequence of one or more such oxidation reactions, with formation of a sulfone or sulfoxide which does not function effectively as an inhibitor for ACE.

Such reports of SQ14225 clinical testing as are currently available, some of which refer to the compound under the name "Captopril", suggest that the product is sufficiently stable in the normal gastric and intestinal environments of most patients to be an effective inhibitor for ACE when administered orally. It is not yet clear, however, whether there may be a group of patients for which SQ14225 is substantially ineffective. Because of the high reactivity of the free sulfhydryl group, SQ14225 could readily form mixed disulfides with serum, cellular proteins, peptides or other free sulfhydryl group-containing substances in the gastric or intestinal environments, in addition to the possibility for dimer formation or oxidative degradation reactions. A mixed disulfide with protein may be antigenic and, indeed, occasional allergic reactions have been clinically observed. See Gavras, et al., New England J. Med. 298,991 (1978). Disulfides and oxidative degradation products of SQ14225, if formed, may at best be expected to be largely ineffective as inhibitors. It may be postulated accordingly that dose response to SQ14225 may vary with conditions of administration and among individual patients. Moreover, in at least some patients, unwanted side effects may occur and maintenance of an effective concentration of the inhibitor in the body may be difficult to control.

Thioester compounds generally are thought to be highly reactive in that the thioester linkage is readily hydrolyzable to a sulfhydryl moiety and a carboxylic moiety. Thioesters are accordingly often used as active ester intermediates for acylation under mild conditions. Such groups, as e.g, acetylthio have been used as blocking groups in the above cited Ondetti, et al. patents. Thioester intermediates are also postulated to occur in the biosynthesis of cyclic peptides such as tyrocidin or gramicidin S. See Lipmann, F, in

Accounts Chem. Res. 6, 361 (1973).

Compounds related to SQ14225 have been disclosed by Ondetti, et al., U.S. Patents 4,046,889, 4,052,511, 4,053,651, 4,113,715 and 4,154,840. Of interest are disclosed analogs of SQ14225 having the five-membered heterocyclic ring of proline replaced by a four- or a six-membered ring. The inhibitory potencies of such analogs relative to SQ14225 are not disclosed. Substitution of D-proline for L-proline is reported to drastically reduce inhibitory potency of 3-mercapto-propanoyl amino acids (Cushman, D.W., et al., supra).

To date, the effect of the amino acid to the left of the sulfur in the thioester compounds has not been determined. It is thought that this amino acid functions as an additional recognition site for the enzyme. If this is true, it would be expected that a compound with an amino acid here would be a better inhibitor. Applicants have found that various amino acids are effective and that the hydroxyprolines, proline-L-, and D,L-3,4-dehydroproline, thiazolidine-4-carboxylic acid and L-5-oxo-proline derivatives are all effective anti-hypertensive agents and have high inhibitory potency for ACE.

## SUMMARY OF THE INVENTION

This invention relates to a plurality of methods for preparing compounds having the formula:

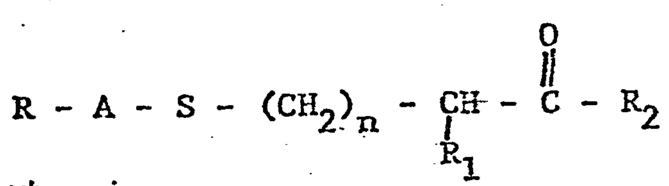

Formula I

wherein

R is hydrogen, formyl, acetyl, propanoyl, butanoyl, phenylacetyl, phenylpropanoyl, benzoyl, cyclopentanecarbonyl, tert-butyloxycarbonyl, cyclopentanecarbonyl-L-lysyl, pyro-L-glutamyl-L-lysyl, L-lysyl, L-arginyl or pyro-L-glutamyl;

A is phenylalanyl, alanyl, trytophyl, tyrosyl, isoleucyl, leucyl, glycyl, histidyl, or valyl, the α-amino group thereof

being in amide linkage with R;

$R_1$ is hydrogen or methyl;

$R_2$ is L-proline, L-3,4-dehydroproline, D,L-3,4-dehydroproline, L-3-hydroxyproline, L-4-hydroxyproline, L-thiazolidine-4-carboxylic acid, or L-5-oxo-proline, the imino group thereof being in imide linkage with the adjacent

$$-\overset{O}{\overset{\|}{C}}-$$

n is 0 or 1, such that when n is 0, $R_1$ is methyl; and where n=1 and $R_1$ is methyl the $-CH_2-\underset{CH_3}{\underset{|}{CH}}-$ moiety is in the D-configuration. These compounds are inhibitors of ACE and are useful as orally effective anti-hypertensive agents.

For convenience, all of the following methods have been described in terms of the steps required to prepare the compound where R is benzoyl, A is phenylalanyl, $R_1$ is methyl, $R_2$ is L-proline and n is 1, i.e. $N^{\alpha}$-[3-$N^{\alpha}$-benzoylphenylalanylthio)-2-D-methylpropanoyl]-L-proline. However, it will be appreciated by those skilled in the art, that other compounds can be made by substituting the desired groups for those used to illustrate the processes where appropriate. For example, L-3,-4-dehydroproline, D,L-3,4-dehydroproline, L-3-hydroxyproline, L-4-hydroxyproline, L-thiazolidine-4-carboxylic acid or L-5-oxo-proline can be substituted for L-proline in all of the methods described below. All of the amino acids listed for A can be similarly substituted for phenylalanine in these methods. Other desired compounds can be made by making the appropriate substitutions unless indicated otherwise below.

In several methods described herein, $N^{\alpha}$-$R_3$-thiophenylalanine where $R_3$ is H, Bz, Boc or Ddz is reacted with methacrylic acid or ester or 3-bromo-2-methylpropionic acid to form 3-$N^{\alpha}$-$R_3$-phenylalanylthio-2-methylpropionic acid or ester. This compound is then resolved to form 3-$N^{\alpha}$-$R_3$-phenylalanylthio-2-D-methylpropionic acid or ester prior to reaction with L-pro to form the desired product. In other

methods $N^{\alpha}$-$R_4$-phenylalanine or $N^{\alpha}$-$R_4$-phenylalanine active ester where $R_4$ is Bz, Bpoc, Ddz is reacted with 3-mercapto-2-methylpropionic acid or ester to form 3-$N^{\alpha}$-$R_4$-phenylalanylthio-2-methylpropionic acid or ester. This compound is then resolved to form 3-$N^{\alpha}$-$R_4$-phenylalanylthio-2-D-methylpropionic acid or ester prior to reaction with L-Pro to form the desired products. In other methods 3-$R_5$-thio-2-methylpropionic acid or ester where $R_5$ is Cbo, acetyl or amino is resolved to form 3-$R_5$-thio-2-D-methylpropionic acid or ester prior to reaction with R-A.

3-benzoylthio-2-D-methylproprionic acid, which is used as a starting material in processes described herein for synthesizing compound of Formula I, is commercially available from Chemical Dynamics Corp., 3001 Hadley Road, South Plainfield, New Jersey.

In the following methods, any conventional coupling methods may be used for those steps requiring them in addition to those listed. Examples of coupling methods include: mixed anhydride (MA), dicyclohexylcarbodiimide (DCC), azide, N-ethyloxycarbonyl-2-ethyloxy-1,2-dihydroquinoline (EEDQ), N-isobutyloxycarbonyl-2-isobutyloxy-1,2-dihydroquinoline, symmetrical anhydride, Woodward's reagent K (N-ethyl-5-phenylisoxazolium-3'-sulfonate), or carbonyldiimidazole (CDI) methods. For a review of these methods see Methoden der Organischen Chemie (Houben-Weyl) Vol. XV, part II, p. 1 et seq. (1974).

Similarly, in the following methods where t-butyloxycarbonyl (Boc) protecting group is used, it can be replaced with any acid sensitive amino protecting group such as 2-(4-biphenyl)-2-propyl)-2-propyloxycarbonyl (Bpoc), 2-phenylisopropyloxycarbonyl(Ppoc), benzyloxycarbonyl (Cbo) or other acid sensitive N-aralkyloxycarbonyl protecting group. Deprotection, i.e. removal of the protecting group, can be effected by any conventional means such as trifluoroacetic acid (TFA) in anisole, HCl in acetic acid, cold trifluoromethane sulfonic acid and methanolic ammonia. See Methoden der Organischen Chemie

(Houben-Weyl) Vol. XV, part I, p. 376 et seq. (1974).

I.

According to a preferredmethod, $N^{\alpha}$-t-butyloxycarbonyl phenylalanine (Boc-Phe) is reacted with thiophenol in the presence of a conventional coupling agent and an anhydrous medium to produce $N^{\alpha}$-t-butyloxycarbonyl-thiophenylalanine phenyl ester. This is done using the MA method in ethyl acetate.

In the second step, the Boc protecting group is removed by reacting the product from step 1 with trifluoroacetic acid (TFA) and anisole. The deprotected product is then reacted with hydrogen chloride in ethanol to produce the hydrogen chloride salt of thiophenylalanine phenyl ester. The protecting group can also be removed with hydrogen chloride or hydrogen bromide in acetic acid or organic solvent, methanesulfonic acid or substituted methanesulfonic acid. This latter compound is then reacted with benzoyl chloride in a mixture of ethyl acetate and water containing $Na_2CO_3$ to produce $N^{\alpha}$-benzoyl-thiophenylalanine phenyl ester.

In the third step, the final product from step 2 is reacted with NaSH in ethanol and in the presence of nitrogen to yield $N^{\alpha}$-benzoyl-thiophenylalanine (Bz-Phe-SH). This step is a modification of Bull. Chem. Soc, Jap. 38, 320 (1965).

Alternatively, the phenyl ester from step 1 is reacted with NaSH as described in step 3 producing Boc-Phe-SH. Or, Boc-Phe-SH can be produced by coupling Boc-Phe with $H_2S$ using the mixed anhydride method. See J. Am. Chem. Soc. 74, 4726 (1952). The benzoyl-thiophenylalanine (Bz-Phe-SH) is then

produced following a procedure similar to that of step 2.

As a second alternative, Bz-Phe-SH can be obtained by reacting benzoyl chloride with Phe in the presence of NaOH. This product is then coupled with $H_2S$ by using the mixed anhydride method. See J. Biol. Chem. 138, 627 (1941).

In the fourth step, this thio acid is heated with methacrylic acid in the presence of toluene to produce 3-($N^{\alpha}$-benzoyl-phenylalanylthio)-2-methylpropionic acid.

The product of this step is then resolved in step 5 to yield $N^{\alpha}$-(3-benzoyl-phenylalanylthio)-2-D-methylpropionic acid by crystallization, liquid chromatography, counter current distribution, ion exchange or by using resolution agents such as 3-nitro-D-tyrosine methyl ester or hydrazide, 3-nitro-D-tyrosine methyl ester or hydrazide, d-(+)-α-methylbenzylamine or l-(-)-α-methylbenzylamine, quinine, (-)-ephedrine or N,N-decyclohexylamine.

In the sixth step, this acid is then coupled with L-proline t-butyl ester by using the DCC or MA methods to produce $N^{\alpha}$-[3-($N^{\alpha}$-benzoyl-phenylalanylthio)-2-D-methylpropanoyl]-L-proline t-butyl ester. This ester is dissolved in a mixture of anisole and TFA to produce $N^{\alpha}$-[3-($N^{\alpha}$-benzoylphenylalanylthio)-2-D-methylpropanoyl]-L-proline. Alternatively, the product from step 5 can be coupled with hydroxysuccinimide using the DCC or MA methods and then the product is reacted with L-proline to yield the same product.

The foregoing method for preparing $N^{\alpha}$-[3-($N^{\alpha}$-benzoyl-phenylalanylthio)-2-D-methylpropanoyl]-L-proline is preferred because the yield is generally high and the product is easily crystallized.

In addition, this method is preferred because the phenylalanyl moiety can be obtained in either the pure L-, D- or D,L- form. That is, by utilizing Boc-L-Phe or Boc-D-Phe as the starting material, $N^{\alpha}$-[3-($N^{\alpha}$-benzoyl-L-phenylalanylthio-2-D-methylpropanoyl]-L-proline or $N^{\alpha}$-[3-($N^{\alpha}$-benzoyl-D-phenylalanylthio)-2-D-methylpropanoyl]-L-proline are obtained. By using a mixture of Boc-L-Phe and Boc-D-Phe, the D,L- form is obtained.

Acrylic acid can be substituted for methacrylic acid in

step 4 above to produce the 3-thio propionic acid derivative.

By substituting the appropriate R-A compound prepared by the methods disclosed in copending U.S. applications Serial Nos. 64,897 through 64,903, filed August 14, 1979, Serial Nos. 116,950 and 116,951, filed January 30, 1980 and Serial No. 121,188 filed March 3, 1980, for Boc-Phe in step 1 and following the procedures of steps 1 and 3-6, a compound having the desired R and A groups can be obtained.

II

According to a second preferred method, 3-benzoylthio-2-D-methylpropionic acid is reacted with 2-methylpropene in the presence of sulfuric acid to yield 3-benzoylthio-2-D-methylpropionic acid t-butyl ester.

In the second step, the benzoyl group is removed by conventional deprotecting methods to produce 3-mercapto-2-D-methylpropionic acid t-butyl ester. Examples of conventional deprotecting methods include using ammonium hydroxide, ammonia in methanol and sodium methoxide in methanol.

In the third step, the product of step 2 is coupled with $N^{\alpha}$-benzoylphenylalanine (Bz-Phe) using the active ester coupling method to form 3-$N^{\alpha}$-benzoylphenylalanylthio-2-D-methylpropionic acid t-butyl ester. Useful active esters include the N-succinimidyl ester, the p-nitrophenyl ester, the pentachlorophenyl ester and the like of Bz-Phe.

Alternatively, the product from step 2 is coupled with Bz-Phe using a low racemization coupling method such as the EEDQ method, the acid azide method or the DCC method with racemization suppressing reagents, e.g., 1-hydroxybenzotriazole, to form 3-$N^{\alpha}$-benzoylphenylalanylthio-2-D-methylpropionic acid t-butyl ester.

In step 4, the t-butyl ester group is removed from the product of step 3 using conventional deprotecting methods to form 3-$N^{\alpha}$-benzoylphenylalanylthio-2-D-methylpropionic acid.

In step 5, the product of step 4 is coupled with N-hydroxysuccinimide using the DCC or MA methods to form the N-succinimidyl ester. This product is then reacted with L-proline to yield $N^{\alpha}$-[3-($N^{\alpha}$-benzoylphenylalanylthio)-2-D-methylpropanoyl]-L-proline. The N-hydroxysuccinimide can be

replaced with other active ester precursors to form other useful active esters. Alternatively, the product of step 4 can be coupled with L-proline t-butyl ester using the DCC or MA methods to produce the t-butyl ester of the same product. The t-butyl ester group is removed using conventional deprotecting methods.

By utilizing Bz-L-Phe, Bz-D-Phe or Bz-D,L-Phe as the starting material, the following compounds can be obtained: $N^{\alpha}$-[3-($N^{\alpha}$-benzoyl-L-phenylalanylthio)-2-D-methylpropanoyl]-L-proline, $N^{\alpha}$-[3-($N^{\alpha}$-benzoyl-D-phenylalanylthio)-2-D-methylpropanoyl]-L-proline or $N^{\alpha}$-[3-$N^{\alpha}$-benzoyl-D,L-phenylalanylthio)-2-D-methylpropanoyl]-L-proline. The pure L-, D-, or D,L-forms of Bz-Phe can be obtained by following the procedures described herein.

By substituting the appropriate R-A compounds prepared by the methods disclosed in copending U.S. Application Serial Nos. 064,897 through 064,903, filed August 14, 1979, Serial Nos. 116,950 and 116,951, filed January 30, 1980 and Serial No. 121,188 filed March 3, 1980 for the Bz-Phe in step 3 and following the procedures of steps 3-5, a compound having the desired R and A groups can be obtained.

III

In a third preferred method, $N^{\alpha}$-Bpoc-Phe is first coupled with 3-mercapto-2-D-methylpropionic acid t-butyl ester (prepared as described in Method II, steps 1-2) using the DCC method in the presence of 1-hydroxybenzotriazole or the MA method to form 3-$N^{\alpha}$-Bpoc-phenylalanylthio-2-D-methylpropionic acid t-butyl ester.

In the second step, the ester produced in step 1 is deprotected to yield 3-$N^{\alpha}$-phenylalanylthio-2-D-methylpropionic acid t-butyl ester.

In the third step, the product of step 2 is coupled to a benzoyl halide, such as benzoyl chloride or bromide, or benzotriazolyl, p-nitrophenyl, o-nitrophenyl, 5-norbornene-2,3-dicarboximidyl, pentachlorophenyl or pentafluorophenyl esters of benzoic acid to yield 3-$N^{\alpha}$-benzoylphenylalanylthio-2-D-methylpropionic acid t-butyl ester.

In the fourth step, the t-butyl ester group is removed

as described in Method II, step 4.

In the fifth step, the product of step 4 is coupled to L proline or L-proline t-butyl ester as described in Method I, step 5 to form $N^{\alpha}$-[3-($N^{\alpha}$-benzoylphenylalanylthio)-2-D-methylpropanoyl]-L-proline.

Any desired A group can be substituted for Phe in step 1 to obtain compounds having the desired A group. Any desired R group can be coupled to the product of step 2 by substituting R for the benzoyl moiety in the benzoyl coupling compounds described in step 3.

In a fourth preferred method, $N^{\alpha}$-t-butyloxycarbonyl-phenylalanine is first coupled to thiophenol, as described in Method I, step 1 above, to produce $N^{\alpha}$-t-butyloxycarbonyl-thiophenylalanine phenyl ester.

In the second step, this ester is then reacted with NaSH in ethanol and in the presence of nitrogen to yield the thio acid, $N^{\alpha}$-t-butyloxycarbonyl-thiophenylalanine. The thio acid can also be prepared by reacting $N^{\alpha}$-t-butyloxycarbonyl-phenylalanine with $H_2S$ as described in Method I.

In the third step, the thio acid is heated with methacrylic acid in toluene to produce 3-($N^{\alpha}$-t-butyloxycarbonyl-phenylalanylthio)-2-methylpropionic acid.

In the fourth step, resolution of this product is accomplished as described in Method I, step 5.

In the fifth step, the resulting product is then deprotected by reaction with a mixture of TFA and anisole. The deprotected product is reacted with benzoyl chloride to produce 3-($N^{\alpha}$-benzoyl-phenylalanylthio)-2-D-methylpropionic acid. $N^{\alpha}$-[3-($N^{\alpha}$-benzoyl-phenylalanylthio)-2-D-methylpropanoyl]-L-proline is produced as described in Method I, step 6.

Alternatively, the product from step 4 can be coupled with L-proline t-butyl ester using the DCC or MA methods. This product is deprotected by a reaction with TFA and anisole. The resulting product is then reacted with benzoyl chloride to produce the desired compound.

As a second alternative, the product from step 4 can be coupled with N-hydroxysuccinimide using the DCC or MA methods.

This product is then reacted with L-proline. The resulting product is deprotected and reacted with benzoyl chloride as previously described to yield the desired product.

As a third alternative, the product from step 2 is deprotected using a mixture of hydrogen chloride in acetic acid to produce the hydrogen chloride salt of Phe-SH. This product is then heated with methacrylic acid and resolved as described in steps 3 and 4. The resulting product is reacted with benzoyl chloride in $Na_2CO_3$ to obtain the desired compound.

Acrylic acid can be substituted for methacrylic acid in step 3 to yield the 3-thiopropionic acid derivative. Any desired R-A compound as described above with respect to Method I can be substituted for Boc-Phe in step 1 of this method. In order to achieve a compound having the desired R and A groups, removal of the R group by deprotection in step 5 is unnecessary.

V.

In a fifth preferred method, the benzoyl group is removed from 3-benzoylthio-2-D-methylpropionic acid using conventional deprotecting methods.

In the second step, the product of step 1 is coupled to $N^{\alpha}$-Boc-Phe using the active ester coupling method as described in Method II, step 3 to form 3-$N^{\alpha}$-Boc-phenylalanylthio-2-D-methylpropionic acid.

The product from step 2 is deprotected in the third step using trifluoroacetic acid (TFA) in anisole to produce the TFA salt of 3-phenylalanylthio-2-D-methylpropionic acid.

In the fourth step, the product from step 3 is reacted with benzoyl chloride in a $NaHCO_3$ solution to form 3-$N^{\alpha}$-benzoylphenylalanylthio-2-D-methylpropionic acid.

$N^{\alpha}$-[3-($N^{\alpha}$-benzoylphenylalanylthio)-2-D-methylpropanoyl]-L-proline is prepared in the fifth step by following the procedure described in Method II, step 5.

Any desired A group can be substituted for Phe in step 2 to obtain compounds having the desired A group. Any desired R group can be coupled to the product of step 3 by substituting R for the benzoyl moiety of the benzoyl chloride coupling

compound described in step 4.

VI.

In a sixth preferred method, 3-mercapto-2-D-methylpropionic acid is coupled to $N^{\alpha}$-Bpoc-Phe using the active ester coupling method as described in Method II, step 3 to form 3-$N^{\alpha}$-Bpoc-phenylalanylthio-2-D-methylpropionic acid.

In the second step, L-pro t-butyl ester is coupled to the product of step 1 using the DCC method in the presence of 1-hydroxybenzotriazole or the MA method to form $N^{\alpha}$-[3-($N^{\alpha}$-Bpoc-phenylalanylthio)-2-D-methylpropanoyl]-L-proline t-butyl ester.

The Bpoc protecting group is removed in the third step using dilute TFA to produce the TFA salt of $N^{\alpha}$-[3-phenylalanylthio-2-D-methylpropanoyl]-L-proline t-butyl ester.

In the fourth step, the product of step 3 is reacted with benzoyl chloride as described in Method V step 4 to form $N^{\alpha}$-[3-($N^{\alpha}$-benzoylphenylalanylthio)-2-D-methylpropanoyl]-L-proline t-butyl ester.

In the fifth step, the t-butyl ester is removed using conventional deprotecting methods to form $N^{\alpha}$-[3-($N^{\alpha}$-benzoylphenylalanylthio)-2-D-methylpropanoyl]-L-proline.

Any desired A can be substituted for Phe in step 1 to obtain compounds having the desired A group. Any desired R group can be coupled to the product of step 3 by substituting R for the benzoyl moiety of the benzoyl chloride coupling compound described in step 4.

VII.

In a seventh preferred method, 3-$N^{\alpha}$-Boc-phenylalanylthio-2-D-methylpropionic acid, prepared as described in Method V., step 2, is coupled with N-hydroxysuccinimide using the DCC or MA methods and this product is then reacted with L-Pro as described in Method II, step 5 to form $N^{\alpha}$-[3-($N^{\alpha}$-Boc-phenylalanylthio)-2-D-methylpropanoyl]-L-proline.

In the second step, the TFA salt of $N^{\alpha}$-[3-phenylalanylthio-2-D-methylpropanoyl]-L-proline is prepared as described in Method V, step 3.

In the third step, benzoyl chloride is reacted with the product of step 2 as described in Method V, step 4 to form

$N^{\alpha}$-[3-($N^{\alpha}$-benzoylphenylalanylthio)-2-D-methylpropanoyl]-L-proline.

Any desired A can be substituted for Phe in step 1 to obtain compounds having the desired A group. Any desired R group can be coupled to the product of step 2 by substituting R for the benzoyl moiety of the benzoyl chloride coupling compounds described in step 3.

In methods III, V, and VI, it is also possible to obtain the A moiety, e.g. phenylalanyl, in either the pure L-D- or D,L-form. For example, by using Bpoc-L-Phe or Bpoc-D-Phe as the starting material, $N^{\alpha}$-[3-($N^{\alpha}$-benzoyl-L-phenylalanylthio)-2-D-methylpropanoyl]-L-proline or $N^{\alpha}$-[3-($N^{\alpha}$-benzoyl-D-phenylalanylthio)-2-D-methylpropanoyl]-L-proline are obtained. By using a mixture of Boc-L-Phe and Boc-D-Phe, the D,L-form is obtained.

VIII

In an eighth preferred method, Boc-Phe is first reacted with pentachlorophenol using the MA method to yield $N^{\alpha}$-t-butyloxy-carbonyl-phenylalanine pentachlorophenyl ester (Boc-Phe-OPcp). Pentafluorophenol or N-hydroxysuccinimide can be used in place of the pentachlorophenol in this reaction.

In the second step, this ester is reacted with hydrogen chloride or hydrogen bromide in acetic acid to produce the HCl or HBr salt of Phe pentachlorophenyl ester.

In the third step, the salt is reacted with benzoyl chloride in a solution of sodium bicarbonate in ethyl acetate and water to yield Bz-Phe pentachlorophenyl ester (Bz-Phe-OPcp).

In the fourth step, Bz-Phe-OPcp is coupled with 3-mercapto-2-methylpropionic acid to form 3-($N^{\alpha}$-benzoyl-phenylalanylthio)-2-methylpropionic acid using any of the well-known coupling agents.

In the fifth step, this product is resolved using any of the resolution agents described above.

In the sixth step, the resolved product is coupled with L-proline t-butyl ester, preferably using the DCC or MA methods, to yield $N^{\alpha}$-[3-$N^{\alpha}$-benzoyl-phenylalanylthio)-2-D-methylpropanoyl]-L-proline t-butyl ester.

Finally, this ester is dissolved in a mixture of anisole and TFA to produced the desired compound.

3-mercaptopropionic acid or 2-mercaptopropionic acid can be substituted for the 3-mercapto-2-methylpropionic acid in step 4 to produce the 3-thiopropionic acid or 2-thiopropionic acid derivatives. Any desired R-A compound as described above with respect to method I can be substituted for Boc-Phe in step 1 of this method. In order to obtain a compound having the desired R and A groups, steps 2 and 3 are eliminated.

IX.

In a ninth preferred method, $N^{\alpha}$-benzoyl-phenylalanine (Bz-Phe) is reacted in the presence of $H_2S$ using the MA method to produce the $N^{\alpha}$-benzoyl-thiophenylalanine (Bz-Phe-SH).

In the second step, Bz-Phe-SH is reacted with methacrylic acid as described in method I, step 4 above.

The product of step 2 is then resolved in the third step as described in method I, step 5 above.

In the fourth step, the resolved product is coupled with L-proline t-butyl ester and then dissolved in TFA and anisole to produce the desired compound as described in method I, step 6 above. Alternatively, the product of step 3 can be coupled with N-hydroxysuccinimide using the DCC or MA methods and then reacted with L-proline to yield the same product.

Any desired R-A compound as described above with respect to method I can be substituted for Bz-Phe in step 1 of this method to obtain a compound having the desired R and A groups. Acrylic acid can be substituted for methacrylic acid in step 2 to produce the 3-thiopropionic acid derivative.

X.

In a tenth preferred method, methacrylic acid is first coupled with N-hydroxysuccinimide followed by reaction with L-proline to yield 2-methylpropenoyl-L-proline.

Alternatively, methacryloyl chloride is reacted with L-proline to produce 2-methylpropenoyl-L-proline.

As a second alternative to the first step, methacrylic acid is coupled with L-proline t-butyl ester using the MA

method to produce the t-butyl ester of 2-methylpropenoyl-L-proline which is then deprotected with TFA to yield 2-methyl-propenoyl-L-proline.

In the second step, the 2-methylpropenoyl-L-proline prepared by any of these alternatives is heated with $N^{\alpha}$-benzoyl-thiophenylalanine (Bz-Phe-SH) in the presence of toluene to produce the desired compound.

Alternatively, the t-butyl ester of 2-methyl-propenoyl-L-proline can be substituted for the 2-methylpropenoyl-L-proline in step 2 above. The product obtained must then be deprotected with TFA to yield the desired compound.

Acrylic acid can be substituted for methacrylic acid, or alternatively acryloyl chloride can be substituted for methacryloyl chloride, in step 1 to yield the 3-thiopropionic acid derivative. Any desired R-A-SH compound can be substituted for Bz-Phe-SH in step 2 of this method to obtain a compound having the desired R and A groups.

XI.

In a further method, benzoyl chloride is reacted with phenylalanine in a NaOH solution to produce $N^{\alpha}$-benzoyl-phenylalanine (Bz-Phe) as described in J. Biol. Chem. 138, 627-629 (1941).

In the second step, thioacetic acid is reacted with methacrylic acid to yield 3-acetylthio-2-methylpropionic acid. This acid is then dissolved in a mixture of methanol and ammonia to yield 3-mercapto-2-methylpropionic acid.

In the third step, Bz-Phe from step 1 is reacted with excess N-hydroxysuccinimide using the MA method. The resulting product is then reacted with the 3-mercapto-2-methylpropionic acid from step 2 to produce 3-($N^{\alpha}$-benzoyl-phenylalanylthio)-2-methylpropionic acid.

In the fourth step, the product of step 3 is resolved using any of the well-known methods.

In the fifth step, the resolved product of step 4 is reacted with N-hydroxysuccinimide using the MA method. The resulting product is then reacted with L-proline to produce the desired compound.

Alternatively, the t-butyl ester of L-proline can be

substituted for L-proline in step 5 above. If this is done the resulting product must be deprotected, for example with TFA in the presence of anisole, to yield the desired compound.

Acrylic acid can be substituted for methacrylic acid in step 2 of this method to obtain the 3-thiopropionic acid derivative. 2-mercaptopropionic acid can also be substituted for the 3-mercapto-2-methylpropionic acid in step 3 to obtain the 2-thiopropionic acid derivative. Any desired R-A compound as described above can be substituted for Bz-Phe in step 3 of this method to obtain a compound having the desired R and A groups.

XII.

In this method, $N^{\alpha}$-benzoyl-phenylalanine (Bz-Phe) is reacted with thiophenol using the MA method to produce $N^{\alpha}$-benzoyl-thiophenylalanine phenyl ester.

Alternatively, Boc-Phe can be substituted for Bz-Phe in step 1 above. The Boc group must then be deprotected and reacted with benzoyl chloride in a subsequent step to produce $N^{\alpha}$-benzoyl-thiophenylalanine phenyl ester.

As another alternative, N-hydroxysuccinimide can be used in the MA reaction in step 1 above.

In the second step, the product of step 1 is reacted with NaSH to yield $N^{\alpha}$-benzoyl-thiophenylalanine as described in method I, step 3 above.

Alternatively, the product of step 1 can be reacted with $H_2S$ in an alkaline solution to produce $N^{\alpha}$-benzoyl-thiophenylalanine. See Naturwissenschaften, 40, 242-243 (1953).

In the third step, the product of step 2 is reacted with an excess of methacrylic acid to form 3-($N^{\alpha}$-benzoyl-phenylalanylthio)-2-methylpropionic acid.

In the fourth step, the product of step 3 is resolved using any of the well-known methods.

In the fifth step, the resolved product is reacted with N-hydroxysuccinimide followed by reaction with L-proline or the t-butyl ester of L-proline as described in method VI, step 5 and its alternative steps above.

Acrylic acid can be substituted for methacrylic acid in

step 3 to obtain the 3-thiopropionic acid derivative. Any desired R-A compound as described above can be substituted for Bz-Phe in step 1 to obtain a compound having the desired R and A groups.

XIII

In a further method, Boc-Phe is first coupled with thiophenol, as described in method IV, step 1, above to produce $N^{\alpha}$-t-butyloxycarbonyl-thiophenylalanine phenyl ester.

The phenyl ester is then reacted with NaSH or $H_2S$ in an alkaline solution to yield Boc-Phe-SH in the second step, as described in method IV step 2 above.

In the third step, the product of step 2 is reacted with an excess of methacrylic acid to form 3-($N^{\alpha}$-t-butyloxy-carbonyl-phenylalanylthio)-2-methylpropionic acid, as described in method IV step 3 above.

In the fourth step, the product of the third step is resolved using any of the well-known methods such as those described in method I, step 5 above.

In the fifth step, the resolved product of step 4 is reacted with HCl in ethyl acetate. The resulting product is then reacted with benzoyl chloride in a mixture of ethyl acetate and water containing $Na_2CO_3$ to yield 3-($N^{\alpha}$-benzoyl-phenylalanylthio)-2-methylpropionic acid.

In the sixth step, the product of step 5 is reacted with N-hydroxysuccinimide using the MA method followed by reaction with L-proline or the t-butyl ester of L-proline, as described above in method XI, step 5 and its alternative, to produce the desired compound.

Acrylic acid can be substituted for methacrylic acid in step 3 to obtain the 3-thiopropionic acid derivative. Any desired R-A compound as described above can be substituted for Boc-Phe in step 1 to achieve a compound having the desired R and A groups, thereby eliminating step 5.

XIV.

In the first step of this method, phenylalanine is coupled to a benzoyl halide, such as benzoyl chloride or benzoyl bromide, or an active benzoic acid ester, such as the N-succinimidyl, p-nitrophenyl, o-nitrophenyl, benzotriazolyl,

5-norbornene-2,3-dicarboximidyl, pentachlorophenyl or pentafluorophenyl esters, to yield benzoylphenylalanine (Bz-Phe).

In the second step, the Bz-Phe from step 1 is coupled to 3-mercapto-2-methylpropionic acid to produce 3-($N^{\alpha}$-benzoyl-phenylalanylthio)-2-methylpropionic acid.

In the third step, the product of step 2 is resolved using any of the conventional methods described above.

In the fourth step, the resolved product is coupled to the t-butyl ester of L-proline using the DCC or MA method to form $N^{\alpha}$-[3-($N^{\alpha}$-benzoyl-phenylalanylthio)-2-methylpropanoyl]-L-proline t-butyl ester. This ester is then deprotected with TFA in anisole to yield the desired compound.

3-mercaptopropionic acid or 2-mercaptopropionic acid can be substituted for the 3-mercapto-2-methylpropionic acid in step 4 to produce the 3-thiopropionic acid and 2-thiopropionic acid derivatives. Any desired R-A compound as described above can be substituted for Bz-Phe in step 2 of this method to obtain a compound having the desired R and A groups.

XV

In another method, N-α,α-dimethyl-3,5-dimethyloxy-benzyloxycarbonyl-phenylalanine (Ddz-Phe) is coupled to 3-mercapto-2-methylpropionic acid using the CDI method in the presence of triethanol amine (TEA) to produce 3-(Ddz-phenyl-alanylthio)-2-methylpropionic acid. Photosensitive amino protecting groups other than Ddz, such as 6-nitroveratryloxy-carbonyl or 2-nitrobenzyloxycarbonyl, may also be used.

In the second step, the product of step 1 is resolved using any of the well-known methods described above.

In the third step, the resolved product of step 2 is coupled to the t-butyl ester of L-proline using the DCC or MA methods to yield $N^{\alpha}$-[3-($N^{\alpha}$-Ddz-phenylalanylthio)-2-methyl-propanoyl]-L-proline t-butyl ester.

In the fourth step, the product of step 3 is deprotected by irradiation to yield $N^{\alpha}$-[3-phenylalanylthio-2-methyl-propanoyl]-L-proline t-butyl ester.

In the fifth step, the product of step 4 is coupled to any of the benzoyl coupling compounds described in method XIV

step 1 above to produce $N^{\alpha}$-[3-($N^{\alpha}$-benzoyl-phenylalanylthio-2-methylpropanoyl]-L-proline t-butyl ester.

In the sixth step, the ester produced in step 5 is deprotected using TFA in anisole to yield the desired compound.

3-mercaptopropionic acid and 2-mercaptopropionic acid can be substituted for 3-mercapto-2-methylpropionic acid in step 1 to obtain the 3-thiopropionic acid and 2-thiopropionic acid derivatives. Any desired group can be substituted for phenylalanine in step 1 to obtain compounds having the desired A group. Any desired R group can be coupled to the product of step 4 by substituting R for the benzoyl group in the benzoyl coupling compounds described in step 1 of method IX which are used in step 5 of this method.

XVI

In a further method, 2-(4-biphenyl)-2-propyloxycarbonyl phenylalanine (Bpoc-Phe) is coupled to 3-mercapto-2-methylpropionic acid using the CDI method as described in method XV, step 1 above. Bpoc can be replaced by 2-phenylisopropyloxycarbonyl (Ppoc) or any other acid sensitive N-aralkyloxycarbonyl protecting group.

In the second step, the 3-($N^{\alpha}$-Bpoc-phenylalanylthio)-2-methylpropionic acid produced in step 1 is resolved using any well-known method of resolution.

In the third step, the resolved product of step 2 is coupled to L-proline t-butyl ester as described above in method XIV, step 3, to produce $N^{\alpha}$-[3-($N^{\alpha}$-Bpoc-phenylalanylthio)-2-methylpropanoyl]-L-proline t-butyl ester.

In the fourth step, the ester produced in step 3 is deprotected with dilute TFA in dichloromethane to yield $N^{\alpha}$-[3-(phenylalanylthio)-2-methylpropanoyl]-L-proline t-butyl ester.

In the fifth step, the product of step 4 is coupled to a benzoyl compound such as that described in method XIV, step 1 above.

In the final step, the product of step 5 is deprotected with TFA in anisole to yield the desired compound.

3-mercaptopropionic acid and 2-mercaptopropionic acid

can be substituted for 3-mercapto-2-methylpropionic acid in step 1 to obtain the 3-thiopropionic acid and 2-thiopropionic acid derivatives. Any desired A group can be substituted for phenylalanine in step 1 to obtain compounds having the desired A group. Any desired R group can be coupled to the product of step 4 by substituting R for the benzoyl group in the benzoyl coupling compounds described in step 1 of method XIV which are used in step 5 of this method.

XVII

In this further method, 3-acetylthio-2-methylpropionic acid is reacted with 2-methylpropene in the presence of sulfonic acid to yield 3-acetylthio-2-methylpropionic acid t-butyl ester. The acetyl group is then removed by dissolving the ester in an alkaline solution to produce 3-mercapto-2-methylpropionic acid t-butyl ester.

In the second step, the product of step 1 is coupled with $N^{\alpha}$-benzoyl-phenylalanine (Bz-Phe) using any of the conventional coupling methods except halide to produce 3-($N^{\alpha}$-benzoyl-phenylalanylthio)-2-methylpropionic acid t-butyl ester.

In the third step, the product of step 2 is deprotected using TFA in anisole to yield 3-($N^{\alpha}$-benzoyl-phenylalanylthio)-2-methylpropionic acid.

In the fourth step, the acid produced in step 3 is resolved using any of the well-known methods described above.

In the fifth step, the resolved product of step 4 is coupled to the t-butyl ester of L-proline, and the resulting ester is then deprotected with TFA in anisole to produce the desired compound as described in method XIV, step 4 above.

3-acetylthiopropionic acid or 2-acetylthiopropionic acid can be substituted for 3-acetylthio-2-methylpropionic acid in step 1 of this method to obtain the 3-thiopropionic acid and 2-thiopropionic acid derivatives. Any desired R-A compound as described above can be substituted for Bz-Phe in step 2 to obtain a compound having the desired R and A groups.

XVIII

0038117

In another method, 3-acetylthio-2-methylpropionic acid is reacted with 2-methyl propene to produce 3-acetylthio-2-methylpropionic acid t-butyl ester followed by removal of the acetyl group to yield 3-mercapto-2-methylpropionic acid t-butyl ester as described in method XVII, step 1 above.

In the second step, the ester produced in step 1 is coupled to a protected phenylalanine compound in which the protecting group is Bpoc, Ppoc or Ddz to produce protected 3-($N^{\alpha}$-phenylalanylthio)-2-methylpropionic acid t-butyl ester.

In the third step, the ester of step 2 is deprotected, for example, using weak acids to yield 3-phenylalanylthio-2-methylpropionic acid t-butyl ester. The Ddz protecting group can be removed using the irradiation method.

In the fourth step, the ester of step 3 is coupled to benzoic acid to produce 3-($N^{\alpha}$-benzoyl-phenylalanylthio)-2-methyl propionic acid t-butyl ester. Any of the benzoyl coupling compounds described in method XIV, step 1 above can be used in place of the benzoic acid.

In the fifth step, the product of step 4 is deprotected with TFA in anisole to yield 3-($N^{\alpha}$-benzoyl-phenylalanylthio)-2-methylpropionic acid as described in method XVII, step 3 above.

In the sixth step, the product of step 5 is resolved using any of the conventional methods described above.

In the seventh step, the resolved product of step 6 is coupled to the t-butyl ester of L-proline and the resulting ester is then deprotected with TFA in anisole, as described in method XIV step 4 above, to yield the desired compound.

In the above steps, the benzyl esters can be substituted for the t-butyl esters and deprotection can be accomplished using HBr in acetic acid.

3-acetylthiopropionic acid or 2-acetylthiopropionic acid can be substituted for 3-acetylthio-2-methylpropionic acid in step 1 to obtain the 3-thiopropionic acid and 2-thiopropionic acid derivatives. Any desired A group can be substituted for phenylalanine in step 2 to obtain a compound having the desired A group. Any desired R group can be coupled to the product of step 3 by substituting R for the

benzoyl group in the benzoyl coupling compound used in step 4.

XIX

In yet another method, Ddz-Phe is coupled to p-nitrophenol using the DCC method to produce $N^{\alpha}$-Ddz-phenylalanine p-nitrophenol ester. The protecting group Ddz can be replaced by Bpoc, Ppoc or Bz. The p-nitrophenol ester group can be replaced by any other active ester group such as those described in method XIV, step 1. The $N^{\alpha}$-Ddz-phenylalanine p-nitrophenol ester is then reacted with NaSH to yield Ddz-thiophenylalanine.

In the second step, the product of step 1 is reacted with methacrylic acid to produce 3-($N^{\alpha}$-Ddz-phenylalanylthio)-2-methylpropionic acid.

In the third step, the acid produced in step 2 is resolved using any conventional resolution method.

In the fourth step, the resolved product of step 3 is coupled to L-proline t-butyl ester, as described in method XV step 3 above, to yield $N^{\alpha}$-[3-($N^{\alpha}$-Ddz-phenylalanylthio-2-methylpropanoyl]-L-proline t-butyl ester.

In the fifth step, the ester of step 4 is deprotected by irradiation to yield $N^{\alpha}$-[3-phenylalanylthio-2-methylpropanoyl]-L-proline t-butyl ester as described above in method XV, step 4.

In the sixth step, the product of step 5 is coupled to any of the benzoyl coupling compounds described in step 1 of method XIV above to produce $N^{\alpha}$-[3-($N^{\alpha}$-benzoyl-phenylalanylthio)-2-methylpropanoyl]-L-proline t-butyl ester as described in method XV, step 5 above.

In the seventh step, the ester produced in step 6 is deprotected with TFA in anisole to yield the desired compound.

Acrylic acid can be substituted for methacrylic acid in step 2 of this method to obtain the 3-thiopropionic acid derivative. Any desired A group can be substituted for phenylalanine in step 1 to obtain a compound having the desired A group. Any desired R group can be coupled to the product of step 5 by substituting R for the benzoyl group in the benzoyl coupling compound used in step 6.

XX

In a further method, methacrylic acid is reacted with L-proline t-butyl ester using the DCC method to produce 2-methylpropenoyl-L-proline t-butyl ester.

In the second step, Ddz-phenylalanine is coupled to p-nitrophenol using the DCC method to produce Ddz-phenylalanine p-nitrophenol ester followed by reaction of this ester with NaSH to yield Ddz-thiophenylalanine as described in method XIX, step 1 above.

In the third step, the product of step 1 is reacted with the product of step 2 to produce $N^{\alpha}$-[3-($N^{\alpha}$-Ddz-phenylalanylthio)-2-methylpropanoyl]-L-proline t-butyl ester.

In the fourth step, the product of step 3 is deprotected by irradiaton to produce $N^{\alpha}$-[3-phenylalanylthio-2-methylpropanoyl]-L-proline t-butyl ester as described in method XV, step 4 above.

In the fifth step, the product of step 4 is coupled to any of the benzoyl coupling compounds described in method XIX, step 1 above to produce $N^{\alpha}$-[3-($N^{\alpha}$-benzoyl-phenylalanylthio)-2-methylpropanoyl]-L-proline t-butyl ester as described in method XV, step 5 above.

In the sixth step, the ester produced in step 5 is deprotected with TFA in anisole.

In the seventh step, the product of step 6 is resolved using any of the conventional methods to yield the desired compound.

As an alternative, $N^{\alpha}$-benzoyl-thiophenylalanine can be substituted for Ddz-thiophenylalanine in step 3, thus eliminating the need for steps 4 and 5.

Acrylic acid can be substituted for methacrylic acid in step 1 of this method to obtain the propenoyl-L-proline derivative. Any desired A group can be substituted for phenylalanine in step 2 to produce a compound having the desired A group. Any desired R group can be coupled to the product of step 4 by substituting R for the benzoyl group in the benzoyl coupling compound used in step 5.

XXI

In this method, 3-acetylthio-2-methylpropionic acid is coupled to L-proline t-butyl ester preferably using the DCC

0038117

method to produce $N^{\alpha}$-(3-acetylthio-2-methylpropanoyl)-L-proline t-butyl ester.

In the second step, the product of step 1 is dissolved in an alkaline solution, such as a mixture of methanol and ammonia, to yield $N^{\alpha}$-(3-mercapto-2-methylpropanoyl)-L-proline t-butyl ester.

In the third step, the product of step 2 is resolved, for example, by using a metal chelate complex.

In the fourth step, the resolved product is coupled to $N^{\alpha}$-benzoyl-phenylalanine (Bz-Phe) to yield $N^{\alpha}$-[3-($N^{\alpha}$-benzoyl-phenylalanylthio-2-D-methylpropanoyl]-L-proline t-butyl ester.

In the fifth step, the product of step 4 is deprotected with TFA in anisole to yield the desired compound as described in method XV, step 6 above.

3-acetylthiopropionic acid or 2-acetylthiopropionic acid can be substituted for 3-acetylthio-2-methylpropionic acid in step 1 of this method to obtain the 3-thiopropionic acid or 2-thiopropionic acid derivatives. Any desired R-A compound as described above can be substituted for Bz-Phe in step 4 to obtain a compound having the desired R and A groups.

XXII

In another method, the desired compound is synthesized using conventional solid phase techniques. See Advan. Enzymol., 32, 221 (1969). An o-nitrobenzyl resin or a chloromethylated resin can be used as the solid phase.

In the first step, Boc-Pro is coupled to the resin. The proline is then deprotected using TFA.

In the second step, 3-($N^{\alpha}$-benzoyl-phenylalanylthio)-2-D-methylpropionic acid is coupled to the proline resin complex produced in step 1 using the DCC method.

In the third step, the desired compound is cleaved from the resin. If o-nitrobenzyl resin is utilized, cleavage is accomplished by irradiation. If a chloromethylated resin is utilized, cleavage is accomplished by using HF.

Any desired compound can be obtained by substituting 3-R-A-S-2-methylpropionic acid, 2-R-A-S-propionic acid or 3-R-A-S-propionic acid for 3-$N^{\alpha}$-benzoyl-phenylalanylthio)-2-

D-methylpropionic acid in step 2.

XXIII

In a further method using a solid phase technique, Boc-Pro is coupled to a solid phase, such as o-nitrobenzyl resin, and then deprotected as described in step 1 of method XXII above.

In the second step, 3-Cbo-thio-2-methylpropionic acid is coupled to the proline resin complex of step 1 using the DCC method. The acetyl group is then removed by treatment with an alkaline solution, such as methanol and ammonia.

In the third step, protected phenylalanine is coupled to the product of step 2. The phenylalanine is protected with Boc, Bpoc, Ppoc or any other acid sensitive protecting group.

In the fourth step, the product of step 3 is deprotected using TFA.

In the fifth step, the product of step 4 is coupled to any of the benzoyl coupling compounds described in step 3 of method XV above.

In the sixth step, $N^{\alpha}$-[3-($N^{\alpha}$-benzoyl-phenylalanylthio)-2-methylpropanoyl]-L-proline is cleaved from the resin as described in method XXII, step 3 above.

In the seventh step, the product of step 6 is resolved using well-known methods to yield the desired compound.

$N^{\alpha}$-benzoyl-phenylalanine can be substituted for the protected phenylalanine compound used in step 3. If this is done, steps 4 and 5 can be eliminated.

3-acetylthiopropionic acid or 2-acetylthiopropionic acid can be substituted for 3-acetylthio-2-methylpropionic acid in step 2 to obtain the 3-thiopropionic acid or 2-thiopropionic acid derivatives. Any desired A group can be substituted for phenylalanine in step 3 to obtain a compound having the desired A group. Any desired R group can be coupled to the product of step 4 by substituting R for the benzoyl group in the benzoyl coupling compound used in step 5.

XXIV

In this method, thiophenylalanine phenyl esterhydrochloride is first reacted with $H_2S$ in a slightly alkaline medium to produce thiophenylalanine hydrochloride.

In the second step, thiophenylalanine hydrochloride is reacted with methacrylic acid to form 3-phenylalanylthio-2-methylpropionic acid hydrochloride.

In the third step, the product of step 2 is resolved using any conventional resolution method.

In the fourth step, the resolved product of step 3 is reacted with any of the benzoyl coupling compounds described in step 1 of method XIV above, such as the N-succinimidyl ester of benzoic acid or benzoyl chloride, to produce 3-($N^{\alpha}$-benzoyl-phenylalanylthio)-2-D-methylpropionic acid.

In the fifth step, the product of step 4 is coupled to the t-butyl ester of L-proline and then deprotected using TFA in anisole to yield the desired compound as described in step 4 of method XIV above.

Steps 3 and 4 can be reversed in this method if desired.

Acrylic acid can be substituted for methacrylic acid in step 2 to obtain the 3-thiopropionic acid derivative. Any desired A group can be substituted for phenylalanine in step 1 to obtain a compound having the desired A group. Any desired R group can be coupled to the product of step 3 by substituting R for the benzoyl group in the benzoyl coupling compound used in step 4.

XXV

In yet another method, benzoyl-thiophenylalanine (Bz-Phe-SH) is reacted with 3-bromo-2-methylpropionic acid to yield 3-($N^{\alpha}$-benzoyl-phenylalanylthio)-2-methylpropionic acid. Chloride or iodine can be substituted for bromine in this reaction.

In the second step, the product of step 1 is coupled to the t-butyl ester of L-proline followed by deprotection with TFA in anisole to yield the desired compound as described above in method XIV, step 4.

By substituting 3-bromopropionic acid or 2-bromopropionic acid for 3-bromo-2-methylpropionic acid in step 1, the 3-thiopropionic acid or 2-thiopropionic acid derivatives can be obtained. By substituting R-A-SH for Bz-Phe-SH in step 1, compounds having the desired R and A groups can be obtained.

XXVI

In a further method, benzyloxycarbonyl mercaptan (Cbo-SH) is reacted with methacrylic acid to produce 3-benzyloxycarbonylthio-2-methylpropionic acid. The Cbo can be replaced with any acid sensitive amino protecting group.

In the second step, the product of step 1 is resolved using any of the well-known resolution methods.

In the third step, the resolved product of step 2 is coupled to the t-butyl ester of L-proline, preferably using the DCC method to produce $N^{\alpha}$-(3-benzyloxycarbonylthio-2-D-methylpropanoyl)-L-proline t-butyl ester.

In the fourth step, the product of step 3 is deprotected with HBr and TFA in anisole to yield 3-mercapto-2-D-methylpropanoyl-L-proline.

In the fifth step, the product of step 4 is coupled to N-benzoyl-phenylalanine (Bz-Phe), preferably using the active ester method, to yield the desired compound.

Acrylic acid can be substituted for methacrylic acid in step 1 to produce the 3-thiopropionic acid derivative. Any desired R-A compound can be substituted for Bz-Phe in step 5 to obtain a compound having the desired R and A groups.

XXVII

In a further method, thiophenylalanine hydrochloride is reacted with methacrylic acid to produce 3-phenylalanylthio-2-methylpropionic acid hydrochloride.

In the second step, the product of step 1 is reacted with benzoyl chloride in TEA to yield 3-($N^{\alpha}$-benzoylphenylalanylthio)-2-methylpropionic acid.

In the third step, the product of step 2 is resolved using any of the conventional resolution methods.

In the fourth step, the resolved product of step 3 is coupled to the t-butyl ester of L-proline and the resulting product is deprotected with TFA in anisole to yield the desired compound as described in step 4 of method XIV above.

Acrylic acid can be substituted for methacrylic acid in step 1 to produce the 3-thiopropionic acid derivative. Any desired A-SH compound can be substituted for thiophenylalanine in step 1 to produce a compound having the desired A group. Any desired R group can be coupled to the product

of step 1 by substituting R for the benzoyl group in benzoyl chloride used in step 2.

XXVIII

In yet a further method, 3-acetylthio-2-methylpropionic acid is reacted with o-nitrobenzyl alcohol to yield 3-acetylthio-2-methylpropionic acid o-nitrobenzyl ester.

In the second step, the ester produced in step 1 is resolved using well-known methods such as those described above.

In the third step, the resolved product of step 2 is deprotected by irradiation to yield 3-acetylthio-2-D-methylpropionic acid.

In the fourth step, the product of step 3 is coupled to the t-butyl ester of L-proline, preferably using the DCC method, to produce $N^{\alpha}$-(3-acetylthio-2-D-methylpropanoyl)-L-proline t-butyl ester.

In the fifth step, the product of step 4 is mixed with an alkaline solution of ammonia, methanol and anisole to produce $N^{\alpha}$-(3-mercapto-2-D-methylpropanoyl)-L-proline t-butyl ester.

In the sixth step, the product of step 5 is coupled to $N^{\alpha}$-benzoyl-phenylalanine (Bz-Phe), preferably using the active ester method, to produce $N^{\alpha}$-[3-($N^{\alpha}$-benzoyl-phenylalanylthio)-2-D-methylpropanoyl]-L-proline t-butyl ester. Other protecting groups such as Boc or Cbo can be substituted for Bz on the phenylalanine used in this reaction.

In the seventh step, the product of step 6 is deprotected, for example with TFA in anisole, to yield the desired compound.

3-acetylthiopropionic acid or 2-acetylthiopropionic acid can be substituted for 3-acetylthio-2-methylpropionic acid in step 1 to yield the 3-thiopropionic acid and 2-thiopropionic acid derivatives. Any R-A compound as described above can be substituted for Bz-Phe in step 6 to obtain a compound having the desired R and A groups.

XXIX

In a further method, 3-amino-2-methylpropionic acid is

first resolved using a conventional method to yield 3-amino-2-D-methylpropionic acid.

In the second step, the product of step 1 is reacted with sodium nitrite in HBr to produce 3-bromo-2-D-methylpropionic acid. Alternatively, HC1 and $HNO_3$ can be used to produce 3-chloro-2-D-methylpropionic acid. As a further alternative, 3-hydroxy-2-methylpropionic acid can be reacted with $PBr_3$ to yield 3-bromo-2-methylpropionic acid.

In the third step, the product of step 2 is reacted with $N^{\alpha}$-benzoyl-thiophenylalanine (Bz-Phe-SH) to produce 3-($N^{\alpha}$-benzoyl-phenylalanylthio)-2-D-methylpropionic acid.

In the fourth step, the product of step 3 is coupled with the t-butyl ester of L-proline and the resulting product is deprotected, for example with TFA in anisole, to yield the desired compound as described in step 4 of method XIV.

3-aminopropionic acid or 2-aminopropionic acid can be substituted for 3-amino-2-D-methylpropionic acid in step 2 to obtain the 3-thiopropionic acid or 2-thiopropionic acid derivatives. Any R-A-SH compound can be substituted for Bz-Phe-SH in step 3 to obtain a compound having the desired R and A groups.

XXX.

In yet another method, 3-amino-2-methylpropionic acid is first resolved using a conventional method to yield 3-amino-2-D-methylpropionic acid as in method XXIX, step 1 above.

In the second step, the product of step 1 is reacted with HBr in sodium nitrite to yield 3-bromo-2-D-methylpropionic acid as described in step 2 of method XXIX above.

In the third step, the product of step 2 is reacted with thiobenzoic acid to produce 3-benzoylthio-2-D-methylpropionic acid. Thioacetic acid may be substituted for thiobenzoic acid in this reaction.

In the fourth step, the product of step 3 is reacted in an alkaline solution, such as methanol and ammonia, to produce 3-mercapto-2-D-methylpropionic acid.

In the fifth step, $N^{\alpha}$-benzoyl-phenylalanine (Bz-Phe) is reacted with N-hydroxysuccinimide using the MA method and the

resulting product is reacted with the product of step 4 to produce 3-($N^{\alpha}$-benzoyl-phenylalanylthio)-2-D-methylpropionic acid as described in step 3 of method XI above.

In the sixth step, the product of step 5 is reacted with N-hydroxysuccinimide using the MA method and the resulting product is reacted with L-proline to produce the desired compound as described above in method XI, step 5.

By substituting 3-aminopropionic acid or 2-aminopropionic acid for 3-amino-2-D-methylpropionic acid in step 2, the 3-thiopropionic acid or 2-thiopropionic acid derivatives can be obtained. Any R-A compound can be substituted for Bz-Phe in step 5 to obtain a compound having the desired R and A groups.

Whenever the D-configuration is indicated for the methyl side chain of the 3-mercapto-2-methylpropionic acid group, it is to be understood that this is equivalent to saying the S-configuration.

The compounds of this invention form basic salts with various inorganic and organic bases which are also within the scope of the invention. Such salts include ammonium salts, alkali metal salts like sodium and potassium salts (which are preferred), alkaline earth metal salts like the calcium and magnesium salts, salts with organic bases, e.g., dicyclohexylamine salt, benzathine, N-methyl-D-glucamine, hydrabamine salts, salts with amino acids like arginine, lysine and the like. The non-toxic, physiologically acceptable salts are preferred, although other salts are also useful, e.g., in isolating or purifying the product, as illustrated in the examples in the case of the dicyclohexylamine salt.

The salts are formed in conventional manner by reacting the free acid form of the product with one or more equivalents of the appropriate base providing the desired cation in a solvent or medium in which the salt is poorly soluble or insoluble, or in water and removing the water by freeze drying. By reacting the salt with an insoluble acid like a cation exchange resin in the hydrogen form (e.g., polystyrene sulfonic acid resin like Dowex 50) or with an aqueous acid and extraction with an organic solvent, e.g., ethyl

acetate, dichloromethane or the like, the free acid form can be obtained, and if desired, another salt formed.

Detailed Description of the Invention

For convenience, details of the methods for preparing $N^{\alpha}$-[3-($N^{\alpha}$-benzoyl-phenylalanylthio)-2-D-methylpropanoyl]-L-proline are described in the following examples. In the following examples, molar equivalents of the reactants were utilized.

I.

Step 1. Synthesis of $N^{\alpha}$-t-butyloxycarbonyl-L-thiophenylalanine phenyl ester.

A solution of 3.98 gm of Boc-L-Phe in approximately 20 ml of redistilled ethanol was cooled to -20°C in a methanol-ice-dry ice bath. To this solution was added 2.04 ml of N-ethylmorpholine followed by 2.04 ml of isobutylchloroformate and the solution was stirred for 3 minutes at -15°C. 1.8 ml of thiophenol was then added and the resulting reaction mixture was stirred for 1 1/2 hours at -15°C. The reaction mixture was slowly warmed to room temperature and then stirred for an additional 2 hours. 20 ml of ethyl acetate was added and the mixture cooled in an ice bath. The organic phase was washed 3 times with cold water, 3 times with cold 1 N citric acid, 3 times with cold saturated NaCl, 3 times with cold 1 N $NaHCO_3$ and finally 3 times with cold saturated NaCl. The organic phase was then dried over anhydrous $MgSO_4$ and filtered. The solvent was removed with a rotary evaporator. The residue was crystallized from benzene-petroleum ether yielding 3.68 gm of white needles having a melting point of 113.5°-114.5°C. Analysis of the product using IR, nmr, elemental analysis, paper electrophoresis and thin layer chromatography, showed the product to be the named compound.

Step 2. Synthesis of $N^{\alpha}$-benzoyl-L-thiophenylalanine phenyl ester.

(i) 2 gm of the product from step 1 was added to 2.0 ml of anisole and cooled in an ice-acetone bath. The $N^{\alpha}$-Boc group was removed by stirring the mixture with 4 ml of anhydrous TFA at room temperature for 30 minutes. TFA was removed at 30° with a rotary evaporator. The TFA and anisole were removed at 30°C with an evaporator under high

vacuum until white crystals first appeared.

(ii) Then 10 ml of 5.7 M hydrogen chloride in ethanol was added and the mixture stirred for 10 minutes at room temperature. 15 ml of anhydrous ether was added and the mixture was cooled in an ice bath for 30 minutes. The solution was filtered and the crystals washed several times with ether. The crystals were dried over night in a vacuum desiccator over NaOH pellets and $P_2O_5$ yielding 1.03 gm of white crystals having a decomposition point of 164.5°-165°C.

(iii) 1.12 gm of this product--hydrogen chloride salt of L-thiophenylalanine phenyl ester-- was suspended in 15 ml of ethyl acetate with vigorous stirring. Then 0.45 ml of benzoyl chloride at room temperature was added. To this mixture was added drop-wise a solution of 1.01 gm of $Na_2CO^3$ in 10 ml of water and the resulting mixture stirred vigorously for another 30 minutes. The mixture was then cooled in an ice bath for 30 minutes and filtered. The residue was washed seven times with cold water, once with cold ethyl acetate and twice with anhydrous ether. The residue was then dried in a vacuum desiccator over NaOH pellets and $P_2O_5$ for several hours yielding 1.32 gm of white crystals having a decomposition point of 182°-183°C. Analysis of the product using IR, nmr, elemental analysis, paper electrophoresis and thin layer chromatography showed the product to be the named compound.

Step 3. Synthesis of $N^{\alpha}$-benzoyl-L-thiophenylalanine.

A stream of nitrogen was bubbled into a solution of 1.01 gm of NaSH in 25 ml of anhydrous ethanol with vigorous stirring and with the temperature maintained at 35°-40°C. 2.17 gm of the product from step 2 was added over a period of 15 minutes. The temperature of the reaction mixture dropped to approximately 20°C by the end of the addition. The mixture was stirred vigorously at 20°C for 1 1/2 hours. The ethanol was removed with a rotary evaporator at 32°C. 30 ml of water was added and the solution cooled in an ice bath. The solution was then acidified to pH 2 using 50% $H_2SO_4$. The mixture was allowed to stand in the ice bath for 30 minutes and then

filtered. The residue was washed several times with cold water and dried over night in a vacuum desiccator over NaOH pellets and $P_2O_5$. The residue was recrystallized from benzene-hexane yielding 1.45 gm of white needles having a decomposition point of 108.5°-109.5°C. Analysis of the product using IR, nmr, elemental analysis, paper electrophoresis and thin layer chromatography showed the product to be the named product.

Step 4. Synthesis of 3-($N^{\alpha}$-benzoyl-L-phenylalanylthio)-2-methylpropionic acid.

A suspension of 285 mg of the product from step 3 and 86.1 ul of methacrylic acid in 2 ml of toluene and was refluxed for several hours until the reaction was virtually completed as indicated by thin layer chromatography and paper electrophoresis. The mixture was then stored overnight at room temperature. Solvent was removed with a rotary evaporator under high vacuum at 35°C yielding an oily residue. The residue was crystallized and recrystallized from benzene-n-hexane yielding 195 mg of white crystals having a melting point of 120°-122.5°C. Analysis of the product by IR, nmr, elemental analysis, paper electrophoresis and thin layer chromatography showed the product to be the named compound.

Step 5. Resolution to yield 3-($N^{\alpha}$-benzoyl-L-phenylalanylthio)-2-D-methylpropionic acid.

(A) The product from step 4 is repeatedly crystallized from benzene to yield the named compound. By utilizing a seed crystal prepared according to the procedure described in Method XXIX steps 1-3 in the repeated crystallization procedure, the named compound is obtained more rapidly and in higher yield.

(B) The product from step 4 is dissolved in benzene. One equivalent of dicyclohexylamine is added to this solution and stirred for several hours. The mixture is let stand overnight at 4°C and then filtered. The crystals are washed and then dried in a vacuum desiccator. The crystals are dissolved in a mixture of water and ethyl acetate. The mixture is acidified to pH 2 with concentrated HCl in an ice

bath. The mixture is then washed with a solution of saturated NaCl and dried with $MgSO_4$. The solution is filtered and the solvent removed with a rotary evaporator. The named product is then crystallized from benzene.

Step 6. Synthesis of $N^{\alpha}$-[3-($N^{\alpha}$-benzoyl-L-phenylalanylthio)-2-D-methyl-propanoyl]-L-proline.

(A) (i) 2.5 mmoles of the product from step 5 is dissolved in 3 ml of dimethylformamide (DMF) and cooled to 0°C in an ice-acetone bath. 2.5 mmoles of DCC in 1.5 ml of DMF is added and the mixture stirred for 5 minutes at 0°C. To this solution is added 2.5 mmoles of L-proline t-butyl ester in 1.5 ml of DMF. The reaction mixture is stirred overnight at 4°C. The reaction mixture is worked-up by addition of 10 ml of ethyl acetate and filtration. The precipitate is washed with 20 ml of ethyl acetate. The combined ethyl acetate fractions are chilled in a freezer. The organic phase is washed three times with saturated NaCl, once with cold 0.1 N $NaHCO_3$ and three times with saturated NaCl. The organic phase is dried with anhydrous $MgSO_4$ and filtered. Solvent is removed with a rotary evaporator under high vacuum yielding a white residue. (ii) The residue is suspended in 4 ml of anisole and reacted with 8 ml of TFA for 1 1/2 hours at room temperature with stirring. TFA is removed with a rotary evaporator under high vacuum. The residue is dissolved in 1 ml of tetrahydrofuran (THF) and chromatographed. The desired fractions are evaporated to dryness, dissolved in a small amount of isopropanol and rechromatographed. The desired fractions yielding an oil are dissolved in THF and then the THF is evaporated under a stream of $N_2$. The final product is obtained after removing the remaining solvent with a rotary evaporator under high vacuum.

(B) A cool solution of 2.5 mmoles of DCC in DMF is added drop-wise to a mixture of 2.5 mmoles of the product from step 5 and 2.5 mmoles of N-hydroxysuccinimide in DMF at 0°C. The reaction mixture is stirred for 30 minutes at 0°C and then overnight at 4°C. Crystalline dicyclohexylurea is removed by filtration and the precipitate washed with ethyl

acetate. Solvents from the combined filtrates are removed under reduced pressure and the residue crystallized from benzene-hexane. The crystals are dissolved in cold THF and then this solution is added to a cold solution of 2.5 mmoles of L-Pro and 2.5 mmoles of $NaHCO_3$ in THF/water. The reaction mixture is stirred overnight at room temperature. The THF is removed with a rotary evaporator at 35°C. Water is added to the mixture and the pH is adjusted to 9 using solid $NaHCO_3$. The aqueous phase is extracted with ethyl acetate. The aqueous phase is cooled in an ice bath and then acidified to pH 2 using 1 N HC1 in the presence of ethyl acetate. The organic phase is washed twice with cold water and then twice with saturated NaC1. The organic phase is dried over anhydrous $MgSO_4$ and filtered. The solvent is removed with a rotary evaporator and the residue is crystallized from ether-hexane yielding the named product.

Alternative Procedures for Synthesizing $N^{\alpha}$-benzoyl-L-thiophenylalanine.

(A) Boc-L-thiophenylalanine phenyl ester is prepared as described in step 1. The thio acid, Boc-L-thiophenylalanine, is prepared by substantially following the procedure described in step 3. Alternatively, this thio acid is prepared by reacting molar equivalents of Boc-L-Phe and $H_2S$ using the mixed anhydride coupling method as described in J. Am. Chem. Soc. 74, 4726 (1952). The thio acid is reacted with hydrogen chloride in ethanol followed by reacting the product with benzoyl chloride or an active ester of benzoic acid as described in step 2 to yield the named product.

(B) $N^{\alpha}$-benzoyl-L-Phe is synthesized by reacting molar equivalents of benzoyl chloride and L-Phe in a sodium hydroxide solution as described in J. Bio. Chem. 138, 627 (1941). The named product is synthesized by reacting molar equivalents of $N^{\alpha}$-benzoyl-L-Phe and $H_2S$ using the mixed anhydride coupling method substantially as described in J. Am. Chem. Soc. 74, 4726 (1952).

Alternative Procedure for Synthesizing 3-($N^{\alpha}$-benzoyl-L-phenylalanylthio)-2-D-methylpropionic acid.

Boc-L-thiophenylalanine is prepared as described in

Method IX, steps 1 and 2. The hydrogen chloride salt of L-thiophenylalanine is prepared by reacting the Boc-L-thiophenylalanine with HCl in acetic acid with stirring at room temperature. Anhydrous ether is then added and the mixture cooled in an ice bath. The solution is filtered and washed several times with ether. The residue is dried overnight in a vacuum desiccator over NaOH pellets and $P_2O_5$. The resulting product is reacted with methacrylic acid as described in step 4 to yield the hydrogen chloride salt of 3-L-phenylalanylthio-2-methylpropionic acid. This product can either be resolved by substantially following the procedure of step 5 and then reacted with benzoyl chloride substantially as described in step 2(iii) to yield the named compound; or, this product can be reacted with benzoyl chloride substantially as described in step 2(iii) and then resolved.

II

Step 1. Synthesis of $N^{\alpha}$-Boc-L-thiophenylalanine phenyl ester.

The named compound was prepared by the procedure described in Method I, step 1.

Step 2. Synthesis of $N^{\alpha}$-Boc-L-thiophenylalanine

The named compound is prepared by substituting the product from step 1 for the $N^{\alpha}$-benzoyl-L-thiophenylalanine phenyl ester in Method I, step 3 and substantially following the procedure described therein. Alternatively, the named compound can be prepared by following the procedure described in J. Am. Chem. Soc. 74, 4726 (1952).

Step 3. Synthesis of 3-($N^{\alpha}$-Boc-L-phenylalanylthio)-2-methylpropionic acid.

The named compound is prepared by substituting the product from step 2 for the $N^{\alpha}$-benzoyl-L-thiophenylalanine in Method I, step 4 and substantially following the procedure described therein.

Step 4. Resolution to yield 3-($N^{\alpha}$-Boc-L-phenylalanylthio)-2-D-methylpropionic acid.

The named compound is obtained upon resolution of the product of step 3 by substantially following the procedure described in Method I, step 5.

Step 5. Synthesis of $N^{\alpha}$-[3-($N^{\alpha}$-benzoyl-L-phenylalanylthio)-2-D-methyl-propanoyl]-L-proline.

(A) The product from step 4 is deprotected using TFA in anisole substantially following the procedure described in Method I, step 2(i). The resulting product is then reacted with benzoyl chloride substantially following the procedure described in Method I, step 2 (III) to yield 3-($N^{\alpha}$-benzoyl-L-phenylalanylthio)-2-D-methylpropionic acid. The named product can be obtained by following the procedures described in Method I, step 6.

(B) By substituting the product of step 4 for the 3-($N^{\alpha}$-benzoyl-L-phenylalanylthio)-2-D-methylpropionic acid in Method I step 6(A) and substantially following the procedure described therein, $N^{\alpha}$-(3-L-phenylalanylthio-2-D-methylpropanoyl)-L-proline is obtained. This product is then reacted with benzoyl chloride substantially following the procedure described in Method I, step 2 (iii) to yield the named compound.

Alternatively, by substituting the product of step 4 for the 3-($N^{\alpha}$-benzoyl-L-phenylalanylthio)-2-D-methylpropionic acid in Method I, step 6(B) and substantially following the procedure described therein, $N^{\alpha}$-[3-($N^{\alpha}$-Boc-L-phenylalanylthio)-2-D-methylpropanoyl]-2-proline is obtained. This product is deprotected by substantially following the procedure of Method I, step 2(i). The named product is obtained by reacting the resulting product with benzoyl chloride as substantially described in Method I, step 2(iii).

III

Step 1. Synthesis of $N^{\alpha}$-Cbo-l-phenylalanine penthachlorophenyl ester.

A solution of 15 mmoles of Cbo-L-Phe in approximately 60 ml of redistilled ethyl acetate is cooled to -20°C in a methanol-ice-dry ice bath. To this solution is added 15 mmoles N-ethyl morpholine followed by 15 mmoles of isobutylchloroformate and the solution is stirred for several minutes at -15°C. 15 mmoles of pentachlorophenol is added and the resulting reaction mixture is stirred for 1 1/2 hours at -15°C. The reaction mixture is slowly warmed to room temperature and then stirred for an additional 2 hours. 100 ml of ethyl

0038117

acetate is added and the mixture cooled in an ice bath. The organic phase is washed several times with cold water, cold saturated NaCl, cold 1 N $NaHCO_3$ and finally with cold saturated NaCl. The organic phase is then dried over anhydrous $MgSO_4$ and filtered. The solvent is removed with a rotary evaporator. The residue is crystallized from ethyl acetate yielding the named product having a melting point of 153.5°-154°C.

Step 2. Synthesis of the hydrogen bromide salt of L-phenylalanine pentachlorophenyl ester.

The product from step 1 is reacted with hydrogen bromide in acetic acid with stirring at room temperature for 45 minutes. Anhydrous ether is added and the mixture is cooled in an ice bath. The solution is filtered and the precipitate washed several times with ether. The precipitate is dried overnight in a vacuum desiccator over NaOH pellets and $P_2O_5$ to yield the named product having a decomposition point of 178°-18°C.

Step 3. Synthesis of $N^{\alpha}$-benzoyl-L-phenylalanine pentachlorophenyl ester.

By substituting the product from step 2 for the hydrogen chloride salt of L-thiophenylalanine in Method I, step 2(iii) and substantially following the procedure described therein, the named compound is obtained having a melting point of 161°-162°C.

Step 4. Synthesis of 3-($N^{\alpha}$-benzoyl-L-phenylalanylthio)-2-methylpropionic acid.

A solution of 10 mmoles of the product from step 3 in redistilled dioxane is added to a solution of 10 mmoles of 3-mercapto-2-methylpropionic acid in dioxane which is neutralized with N-ethyl morpholine. The reaction mixture is stirred at room temperature until the reaction, as judged by thin layer chromatography, is completed. The solvent is removed under reduced pressure at 30°C and ethyl acetate is added to the residue. The mixture is cooled in an ice bath and washed with 0.1 N HCl and then several times with saturated NaCl. The solvent is removed with a rotary evaporator after drying over anhydrous $MgSO_4$ to yield the named product.

Step 5. Resolution to yield 3-($N^{\alpha}$-benzoyl-L-phenylalanylthio)-2-D-methylpropionic acid.

The named compound is obtained by following the procedure described in Method I, step 5.

Step 6. Synthesis of $N^{\alpha}$-[3-$N^{\alpha}$-benzoyl-L-phenylalanylthio)-2-D-methylpropanoyl]-L-proline.

The named compound is obtained by following the procedure described in Method I, step 6(A).

IV

Step 1. Synthesis of $N^{\alpha}$-benzoyl-D,L-thiophenylalanine.

The named product is synthesized by reacting molar equivalents of $N^{\alpha}$-benzoyl-D,L-Phe and $H_2S$ using the mixed anhydride coupling method substantially as described in J. Am. Chem. Soc. 74, 4726 (1952).

Step 2. Synthesis of 3-($N^{\alpha}$-benzoyl-D,L-phenylalanylthio)-2-methylpropionic acid.

The named product is obtained by substituting the product of step 1 for the $N^{\alpha}$-benzoyl-L-thiophenylalanine of Method I, step 4 and substantially following the procedure therein.

Step 3. Resolution to yield 3-($N^{\alpha}$-benzoyl-D,L-phenylalanylthio)-2-D-methylpropionic acid.

The named product is obtained by substantially following the procedure described in Method I, step 5.

Step 4. Synthesis of $N^{\alpha}$-[3-($N^{\alpha}$-benzoyl-D,L-phenylalanylthio)-2-D-methylpropanoyl]-L-proline.

The named product is obtained by substituting the product from step 3 into Method I, step 6 and substantially following the procedure therein.

V.

Step 1. Synthesis of 2-methyl-propenoyl-L-proline.

(i) A cool solution of 5 mmoles of DCC in dichloromethane is added drop-wise to a mixture of 5 mmoles of methacrylic acid and 5 mmoles of N-hydroxysuccinimide in dichloromethane at 0°C. The reaction mixture is stirred for 30 minutes at 0°C and then overnight at 4°C. Crystalline dicyclohexylurea is removed by filtration and the precipitate washed with ethyl acetate. Solvents from the combined filtrates are removed under reduced pressure and the residue crystallized from benzene-hexane. The crystals are dissolved in cold THF and then this solution is added to a cold solution of 5 mmoles of L-Pro and 5 mmoles of $NaHCO_3$ in THF and water. The reaction mixture is stirred overnight at room temperature. The THF is removed with a rotary evaporator at 35°C. Water is added to the mixture and the pH is adjusted to 9 using solid $NaHCO_3$. The aqueous phase is extracted with ethyl acetate. The aqueous phase is cooled in an ice bath and then acidified to pH 2 using 1 N HCl in the presence of ethyl acetate. The organic phase is washed with cold water and saturated NaCl. The organic phase is dried over anhydrous $MgSO_4$ and filtered. The solvent is removed with a rotary evaporator and the residue is crystallized from ether-hexane yielding the named product.

(ii) 2.5 mmoles of methacryloyl chloride is added to a solution of 2.5 mmoles of L-proline in a mixture of water and sodium bicarbonate chilled in an ice bath with vigorous stirring. When the addition is completed, the mixture is stirred at room temperature for two hours, and then extracted with ether. The aqueous phase is acidified with 1.0 N HCl and extracted with ethyl acetate. The organic phase is concentrated to dryness in vacuo to yield the named product.

(iii) A solution of 2.5 mmoles of methacrylic acid in redistilled ethyl acetate is cooled to -20°C in a methanol-ice-dry ice bath. To this solution was added 2.5 mmoles of N-ethyl morpholine followed by 2.5 mmoles of isobutylchloroformate and the solution is stirred for several minutes at -15°C. 2.5 mmoles of L-proline t-butyl ester is then added and the resulting reaction mixture is stirred for 1 1/2 hours at -15°C. The reaction mixture is slowly warmed to room temperature and then stirred for an additional 2 hours. Ethyl acetate is added and the mixture cooled in an ice bath. The organic phase is washed several times with cold water, cold 1 N citric acid, cold saturated NaCl, cold 1 N $NaHCO_3$ and finally with cold saturated NaCl. The organic phase is dried over anhydrous $MgSO_4$ and filtered. The solvent is removed with a rotary evaporator. The residue is crystallized from

benzene-petroleum ether. The t-butyl ester group is removed by substantially following the description thereof in Method I, step 6(A).

Step 2. Synthesis of $N^{\alpha}$-[3-($N^{\alpha}$-benzoyl-L-phenylalanylthio)-2-D-methylpropanoyl]-L-proline.

The product of step 1 is substituted for the methacrylic acid in Method I, step 4, and the method described therein is substantially followed to yield the 2-D,L-methyl-propanoyl derivative. This derivative is resolved by repeated crystallization from methanol-benzene-ether or by liquid chromatography to yield the named product. Alternatively, $N^{\alpha}$-2-methyl-propenoyl-L-Pro t-butyl ester can be used in place of the product from step 1. After resolution the resulting product is deprotected as described in step 1 to yield the named compound.

VI

Step 1. Synthesis of $N^{\alpha}$-benzoyl-L-phenylalanine.

The named product is synthesized by reacting molar equivalents of benzoyl chloride and L-Phe in a NaOH solution using the method substantially as described in J. Biol. Chem. 138, 627 (1941).

Step 2. Synthesis of 3-mercapto-2-methylpropionic acid.

A mixture of thioacetic acid (50 g) and methacrylic acid (40.7g) is heated on a steam bath for 1 hour and then stored at room temperature for 18 hours. After confirming by nmr spectroscopy that complete reaction of methacrylic acid has been achieved, the reaction mixture is distilled in vacuo and the desired 3-acetylthio-2-methylpropionic acid is separated in the fraction with boiling point 128.5°-131°C and is concentrated to dryness. The product (3.4 g) is dissolved in a mixture of water (10.5 ml) and concentrated ammonia (6.4 ml). After 1 hour the reaction mixture is diluted with water and filtered. The filtrate is extracted with ethyl acetate and then acidified with concentrated HCl, saturated with NaCl and extracted twice with ethyl acetate. The ethyl acetate extracts are washed with saturated NaCl and concentrated to dryness to yield the named product.

Step 3. Synthesis of 3-($N^{\alpha}$-benzoyl-L-phenylalanylthio)-2-methyl-propionic acid.

A solution of 25 mmoles of $N^{\alpha}$-benzoyl-L-Phe in redistilled ethyl acetate is added to -20°C in a methanoyl-ice-dry ice bath. To this solution is added 25 mmoles of N-ethyl morpholine followed by 25 mmoles of isobutylchloroformate and the solution is stirred for several minutes at -15°C. 50 mmoles of N-hydroxysuccinimide acid is added and the resulting reaction mixture stirred for 1 1/2 hours at -15°C. The reaction mixture is slowly warmed to room temperature and 25 mmoles of 3-mercapto-2-methylpropionic acid, neutralized with N-ethyl morpholine, is added and then stirred until the reaction, as judged by thin layer chromatography, is completed. Ethyl acetate is added and the mixture cooled in an ice bath and acidified to pH 3 with 1 N HC1. The organic phase is washed several times with cold water and cold saturated NaC1. The organic phase is then dried over anhydrous $MgSO_4$ and filtered. The solvent is removed with a rotary evaporator. The residue is crystallized from benzene-petroleum ether yielding the named product.

Step 4. Resolution to yield $N^{\alpha}$-[3-($N^{\alpha}$-benzoyl-L-phenylalanylthio)-2-D-methylpropanoyl]-L-proline.

The named product is obtained by following the procedure described in Method I, step 5.

Step 5. Synthesis of $N^{\alpha}$-[3-($N^{\alpha}$-benzoyl-L-phenylalanylthio)-2-D-methylpropanoyl]-L-proline.

A solution of 10 mmoles of the product from step 4 in redistilled ethyl acetate is cooled to -20°C in a methanol-ice-dry ice bath. To this solution is added 10 mmoles of N-ethyl morpholine followed by 10 mmoles of isobutylchloroformate and the solution is stirred for several minutes at -15°C. 10 mmoles of N-hydroxysuccinimide is then added and the resulting reaction mixture stirred for 1 1/2 hours at -15°C. The reaction mixture is slowly warmed to room temperature and then stirred for an additional two hours. Ethyl acetate is added and the mixture cooled in an ice bath. The organic phase is washed several times with cold water, cold

1 N $NaHCO_3$ and finally with cold saturated NaCl. The organic phase is then dried over anhydrous $MgSO_4$ and filtered. The solvent is removed with a rotary evaporator. The residue is crystallized from benzene-petroleum ether to yield the N-hydroxysuccinimide ester of 3-($N^{\alpha}$-benzoyl-L-phenylalanylthio)-2-D-methylpropionic acid. This product is dissolved in THF and then the solution is added to a cold solution of 10 mmoles of L-Pro in THF:water (1:1) containing $NaHCO_3$. The mixture is stirred overnight at room temperature. Ethyl acetate is added and the mixture cooled in an ice bath. The organic phase is washed with cold water, cold 1 N citric acid, cold saturated NaCl, cold 1 N $NaHCO_3$ and finally with cold saturated NaCl. The organic phase is then dried over anhydrous $MgSO_4$ and filtered. The solvent is removed with a rotary evaporator. The residue is crystallized from ether-hexane yielding the named product.

VII

Step 1. Synthesis of $N^{\alpha}$-benzoyl-L-thiophenylalanine phenyl ester.

The named product is obtained by substituting benzoyl-L-phenylalanine for the Boc-L-Phe in Method I, step 1 and substantially following the procedure described therein.

Step 2. Synthesis of $N^{\alpha}$-benzoyl-L-thiophenylalanine.

The named product is obtained by following the procedure described in Method I, step 3 using the product of step 1.

Step 3. Synthesis of 3-($N^{\alpha}$-benzoyl-L-phenylalanylthio)-2-methylpropionic acid.

The named product is obtained by following the procedure described in Method I, step 4 using the product of step 2.

Step 4. Resolution to yield 3-($N^{\alpha}$-benzoyl-L-phenylalanylthio)-2-D-methylpropionic acid.

The named product is obtained by following the procedure described in Method I, step 5.

Step 5. Synthesis of $N^{\alpha}$-[3-($N^{\alpha}$-benzoyl-L-phenylalanylthio)-2-D-methylpropanoyl]-L-proline.

The named product is obtained by following the procedure described in Method XI, step 5 using the product of step 4.

VIII

Step 1. Synthesis of $N^{\alpha}$-Boc-L-thiophenylalanine phenyl ester.

The named product is obtained by following the procedure described in Method I, step 1.

Step 2. Synthesis of $N^{\alpha}$-Boc-L-thiophenylalanine.

The named product is obtained by following the procedure described in Method I, step 3.

Step 3. Synthesis of 3-($N^{\alpha}$-Boc-L-phenylalanylthio)-2-methylpropionic acid.

The named product is obtained by following the procedure described in Method IV, step 3.

Step 4. Resolution to yield 3-($N^{\alpha}$-Boc-L-phenylalanylthio)-2-D-methylpropionic acid.

The named product is obtained by following the procedure described in Method I, step 5.

Step 5. Synthesis of 3-($N^{\alpha}$-benzoyl-L-phenylalanylthio)-2-D-methylpropionic acid.

By substituting the product from step 4 for the $N^{\alpha}$-Boc-L-thio-phenylalanine ester in Method 1, step 2 and substantially following the procedure of Method I, step 2, the named product is obtained.

Step 6. Synthesis of $N^{\alpha}$-[3-($N^{\alpha}$-benzoyl-L-phenylalanylthio)-2-D-methylpropanoyl]-L-proline.

The named compound is synthesized by following the procedure of Method XI, step 5.

IX

Step 1. Synthesis of $N^{\alpha}$-benzoyl-L-phenylalanine.

The named product is synthesized by following the procedure described in Method XI, step 1.

Step 2. Synthesis of 3-($N^{\alpha}$-benzoyl-L-phenylalanylthio)-2-methylpropionic acid.

The named product is synthesized by substantially following the procedure described in Method XI, step 3. Some racemization may occur using this method.

Step 3. Resolution to yield 3-($N^{\alpha}$-benzoyl-L-phenylalanylthio)-2-D-methylpropionic acid.

The named product is obtained by following the procedure described in Method I, step 5.

Step 4. Synthesis of $N^{\alpha}$-[3-($N^{\alpha}$-benzoyl-L-phenylalanylthio)-2-D-methylpropanoyl]-L-proline.

The named product is synthesized by following the

procedure described in Method I, step 6(a).

X

Step 1. Synthesis of 3-($N^{\alpha}$-Ddz-L-phenylalanylthio)-2-methylpropionic acid.

Ddz-L-Phe is synthesized by following the procedure described in Liebigs Ann. Chem. 763, 162 (1972) or in Birr. C., Peptide, 1972, Proc. of the 12th Eur. Pept. Symp. Hanson, H. and Jaknbke, H.D., Ed. North-Holland Publishers, p. 72 (1973). A solution of 10 mmoles of Ddz-L-Phe in redistilled dioxane is cooled in an acetone-ice-dry ice bath at -20°C. To this solution is added 10 mmoles of 1,1-dicarbonyldiimidazole (CDI) in dioxane. The solution is stirred at -10°C for 2 hours and then added to a cold solution of 10 mmoles of 3-mercapto-2-methylpropionic acid in dioxane which is neutralized with N-ethyl morpholine. The reaction is stirred at -10°C until the reaction as judged by thin layer chromatography, is completed and then slowly warmed to room temperature. The solvent is removed under reduced pressure at 30°C and ethyl acetate is added to the residue. The mixture is cooled in an ice bath and washed with 0.1 N HC1 and then several times with saturated NaC1. The solvent is removed with a rotary evaporator after drying over anhydrous $MgSO_4$ to yield the named product.

Step 2. Resolution to yield 3-($N^{\alpha}$-Ddz-L-phenylalanylthio)-2-D-methylproprionic acid.

The named compound is obtained by substantially following the procedure described in Method I, step 5.

Step 3. Synthesis of $N^{\alpha}$-[3-($N^{\alpha}$-Ddz-L-phenylalanylthio)-2-D-methylpropanoyl]-L-proline t-butyl ester.

The named compound is synthesized by substituting the product from step 2 for the 3-($N^{\alpha}$-benzoyl-L-phenylalanylthio)-2-D-methylpropionic acid in Method I, step 6(A)(i) and substantial following the procedure described therein.

Step 4. Synthesis of $N^{\alpha}$-[3-(L-phenylalanylthio)-2-D-methylpropanoyl]-L-proline t-butyl ester.

The product from step 3 is dissolved in THF and cooled to 20°C. The mixture transferred to a vial and irradiated with light of a wavelength 350nm for 5 hours. The THF is removed with a rotary evaporator to yield the named product.

Step 5. Synthesis of $N^{\alpha}$-[3-($N^{\alpha}$-benzoyl-L-phenylalanylthio)-2-D-methylpropanoyl]-L-proline t-butyl ester.

The named compound is synthesized by substituting the product from step 4 in Method I, step 2(iii) and substantially following the procedure described therein.

Step 6. Synthesis of $N^{\alpha}$-[3-($N^{\alpha}$-benzoyl-L-phenylalanylthio)-2-D-methylpropanoyl]-L-proline.

The named product is obtained by removing the t-butyl ester group of the product of step 5 by following the procedure described in Method I, step 6(A)(ii).

XI

Step 1. Synthesis of 3-($N^{\alpha}$-Bpoc-L-phenylalanylthio)-2-methylpropionic acid.

$N^{\alpha}$-Bpoc-L-Phe is synthesized by following the procedure described in Helv. Chem. Acta 51, 614 (1968). The named compound is prepared by substituting $N^{\alpha}$-Bpoc-L-Phe for the Ddz-L-Phe in Method XV, step 1 and substantially following the procedure described therein.

Step 2. Resolution to yield 3-($N^{\alpha}$-Bpoc-L-phenylalanylthio)-2-D-methylpropionic acid.

The named compound is obtained by substantially following the procedure described in Method I, step 5.

Step 3. Synthesis of $N^{\alpha}$-[3-($N^{\alpha}$-Bpoc-L-phenylalanylthio)-2-D-methylpropanoyl]-L-proline-t-butyl ester.

By substituting the product from step 2 for the 3-($N^{\alpha}$-benzoyl-L-phenylalanylthio)-2-D-methylpropionic acid in Method I, step 6(A)(i) and substantially following the procedure described therein, the named product is obtained.

Step 4. Synthesis of $N^{\alpha}$-[3-(L-phenylalanylthio)-2-D-methylpropanoyl]-L-proline t-butyl ester.

The product from step 3 is dissolved in methylene chloride containing 0.5% TFA and stirred for 10 minutes. The TFA and methylene chloride is then removed with a rotary evaporator to yield the named product.

Step 5. Synthesis of $N^{\alpha}$-[3-($N^{\alpha}$-benzoyl-L-phenylalanylthio)-2-D-methylpropanoyl]-L-proline t-butyl ester.

The named product is prepared by substituting the product from step 4 in Method I, step 2(iii) and substantially following the procedure therein.

Step 6. Synthesis of $N^{\alpha}$-[3-($N^{\alpha}$-benzoyl-L-phenylalanylthio)-2-D-methylpropanoyl]-L-proline.

The named product is obtained by removing the t-butyl ester group of the product of step 5 by following the procedure described in Method I, step 6(A)(ii).

XII

Step 1. Synthesis of 3-mercapto-2-methylpropionic acid t-butyl ester.

0.62 gm of 3-acetylthio-2-methylpropionic acid is added to a solution of 0.56 gm of 2-methylpropene in 1 N sulfuric acid at -10°C. The mixture is stirred for 30 minutes and then warmed to room temperature to yield 3-acetylthio-2-methylpropionic acid t-butyl ester. This product is dissolved in a mixture of ammonia and water. After 1 hour the reaction mixture is diluted with water and filtered. The filtrate is extracted with ethyl acetate and then acidified with concentrated HCl, saturated with NaCl and extracted twice with ethyl acetate. The ethyl acetate extracts are washed with saturated NaCl and concentrated to dryness to yield the named product.

Step 2. Synthesis of 3-($N^{\alpha}$-benzoyl-L-phenylalanylthio)-2-methylpropionic acid t-butyl ester.

By substituting the product from step 1 for the 3-mercapto-2-methylpropionic acid in Method XI, step 3 and substantially following the procedure described therein, the named product is obtained.

Step 3. Synthesis of 3-($N^{\alpha}$-benzoyl-L-phenylalanylthio)-2-methylpropionic acid.

The named product is obtained by removing the t-butyl ester group of the product of step 2 by substantially following the procedure in Method I, step 6(A)(ii).

Step 4. Resolution to yield 3-($N^{\alpha}$-benzoyl-L-phenylalanylthio)-2-D-methylpropionic acid.

The named product is obtained by following the procedure described in Method I, step 5.

Step 5. Synthesis of $N^{\alpha}$-[3-(benzoyl-L-phenyl alanylthio)-2-D-methylpropanoyl]-L-proline.

The named product is synthesized by following the procedure described in Method I, step 6(A).

XIII.

Step 1. Synthesis of 3-mercapto-2-methylpropionic acid t-butyl ester.

The named product is synthesized following the procedure described in Method XVII, step 1.

Step 2. Synthesis of 3-($N^{\alpha}$-Ppoc-L-phenylalanylthio)-2-methylpropionic acid t-butyl ester.

By substituting $N^{\alpha}$-Ppoc-L-Phe (which is prepared by following the procedure described in Int. J. Peptide Protein Res. 6, 111 (1974))for the $N^{\alpha}$-Ddz-L-Phe and by substituting the product from step 1 for the 3-mercapto-2-methylpropionic acid in Method XV, step 1 and substantially following the procedure described therein, the named product is obtained.

Step 3. Synthesis of 3-L-phenylalanylthio-2-methylpropionic acid t-butyl ester.

The product from step 2 is dissolved in methylene chloride containing 0.5% TFA and stirred for 10 minutes. The TFA and methylene chloride is then removed with a rotary evaporator to yield the named product.

Step 4. Synthesis of 3-($N^{\alpha}$-benzoyl-L-phenylalanylthio)-2-methylpropionic acid t-butyl ester.

The named product is synthesized by reacting molar equivalents of benzoyl chloride and the product from step 3 in a mixture of ethyl acetate and water containing $NaHCO_3$.

Step 5. Synthesis of 3-($N^{\alpha}$-benzoyl-L-phenylalanylthio)-2-methylpropionic acid.

The named product is obtained by removing the t-butyl ester group of the product of step 4 by substantially following the procedure in Method I, step 6(A)(ii).

Step 6. Resolution to yield 3-($N^{\alpha}$-benzoyl-L-phenylalanylthio)-2-D-methyl-propionic acid.

The named product is obtained by following the procedure described in Method I, step 5.

Step 7. Synthesis of $N^{\alpha}$-[3-($N^{\alpha}$-benzoyl-L-phenylalanylthio)-2-D-methylpropanoyl]-L-proline.

The named product is synthesized by following the procedure described in Method I, step 6(A).

XIV

Step 1. Synthesis of $N^{\alpha}$-Ddz-L-thiophenylalanine.

5 mmoles of $N^{\alpha}$-Ddz-L-Phe is dissolved in DMF and cooled to 0°C in an ice-acetone bath. 5 mmoles of DCC in DMF is added and the mixture is stirred for several minutes at 0°C. To this solution is added 5 mmoles of p-nitrophenol in DMF. The reaction mixture is stirred overnight at 4°C. The solvent is removed by a rotary evaporator. Ethyl acetate is added and the mixture filtered. The residue is washed with ethyl acetate. The combined ethyl acetate fractions are chilled in a freezer. The organic phase is washed three times with cold 1 N $NaHCO_3$ and finally three times with saturated NaC1. The organic phase is dried over anhydrous $MgSO_4$ and filtered. Solvent is removed with a rotary evaporator under high vacuum. By substituting this product for the benzoyl-L-thiophenylalanine phenyl ester of Method I, step 3 and substantially following the procedure described therein, the named product is obtained.

Step 2. Synthesis of 3-($N^{\alpha}$-Ddz-L-phenylalanylthio)-2-methylpropionic acid.

By substituting the product from step 1 for the $N^{\alpha}$-benzoyl-L-thiophenylalanine of Method I, step 4 and substantially following the procedure described therein, the named product is obtained.

Step 3. Resolution to yield 3-($N^{\alpha}$-Ddz-L-phenylalanylthio)-2-D-methylpropionic acid.

The named compound is obtained by substantially following the procedure described in Method I, step 5.

Step 4. Synthesis of N-[3-($N^{\alpha}$-Ddz-L-phenylalanylthio)-2-D-methylpropanoyl]-L-proline t-butyl ester.

The named product is synthesized by substituting the product from step 3 for the 3-($N^{\alpha}$-benzoyl-L-phenylalanylthio)-2-D-methylpropionic acid in Method I, step 6(A)(i) and substantially following the procedure described therein.

Step 5. Synthesis of $N^{\alpha}$-[3-(L-phenyl-alanylthio)-2-D-methylpropanoyl]-L-proline t-butyl ester.

The named compound is synthesized by following the procedure of Method XV, step 4.

Step 6. Synthesis of $N^{\alpha}$-[3-($N^{\alpha}$-benzoyl-L-phenylalanylthio)-2-D-methylpropanoyl]-L-proline t-butyl ester.

The named product is synthesized by reacting molar equivalents of benzoyl chloride and the product from step 5 in a mixture of ethyl acetate and water containing $NaHCO_3$.

Step 7. Synthesis of $N^{\alpha}$-[3-($N^{\alpha}$-benzoyl-L-phenylalanylthio)-2-D-methylpropanoyl]-L-proline.

The named product is synthesized by removing the t-butyl ester group of the product of step 6 by following the procedure described in Method I, step 6(A)(ii).

XV

Step 1. Synthesis of 2-methyl-propenoyl-L-proline t-butyl ester.

A cool solution of 2 mmoles of DCC in DMF is added dropwise to a mixture of 2 mmoles of methacrylic acid and 2 mmoles of L-proline t-butyl ester in DMF at 0°C. The reaction mixture is stirred for 30 minutes and then overnight at 4°C. Crystalline dicyclohexylurea is removed by filtration and the precipitate is washed with ethyl acetate. Solvents from the combined filtrates are removed under pressure and the residue is crystallized from benzene-hexane to yield the named product.

Step 2. Synthesis of $N^{\alpha}$-Ddz-L-thiophenylalanine.

The named compound is prepared following the procedure described in Method XIX, step 1.

Step 3. Synthesis of $N^{\alpha}$-[3-($N^{\alpha}$-Ddz-L-phenylalanylthio)-2-methylpropanoyl]-L-proline t-butyl ester.

By substituting the product of step 2 for the benzoyl-L-thiophenylalanine and the product of step 1 for the methacrylic acid of Method I, step 4 and substantially following the procedure described therein, the named product is obtained.

Step 4. Synthesis of $N^{\alpha}$-[3-(L-phenylalanylthio)-2-methylpropanoyl]-L-proline t-butyl ester.

The named compound is synthesized by following the

procedure of Method XV step 4.

Step 5. Synthesis of $N^{\alpha}$-[3-($N^{\alpha}$-benzoyl-L-phenylalanylthio)-2-methylpropanoyl]-L-proline t-butyl ester.

The named compound is synthesized by following the procedure described in Method XIX, step 6.

Step 6. Synthesis of $N^{\alpha}$-[3-($N^{\alpha}$-benzoyl-L-phenylalanylthio)-2-methylpropanoyl]-L-proline.

The named compound is obtained by removing the t-butyl ester group of the product of step 5 by following the procedure of Method I, step 6(A)(ii).

Step 7. Resolution to yield $N^{\alpha}$-[3-($N^{\alpha}$-benzoyl-L-phenylalanylthio)-2-D-methylpropanoyl]-L-proline.

The named compound is obtained by substantially following the procedure described in Method X step 2.

XVI

Step 1. Synthesis of 3-acetylthio-2-methylpropanoyl-L-proline t-butyl ester.

By substituting 3-acetylthio-2-methylpropionic acid for the methacrylic acid in Method XX, step 1 and substantially following the peocedure described therein, the named compound is obtained.

Step 2. Synthesis of 3-mercapto-2-methyl-propanoyl-L-proline t-butyl ester.

The product from step 1 is dissolved in a mixture of methanol and concentrated ammonia. After 1 hour the reaction mixture is diluted with water and filtered. The filtrate is extracted with ethyl acetate and then acidified with concentracted HCl, saturated with NaCl and extracted twice with ethyl acetate. The ethyl acetate extracts are washed with saturated NaCl and concentrated to dryness yielding the named product.

Step 3. Resolution to yield 3-mercapto-2-D-methylpropanoyl-L-proline t-butyl ester.

The product from step 2 is resolved using liquid chromatography or by forming a metal chelate complex to yield the named product.

Step 4. Synthesis of $N^{\alpha}$-[3-($N^{\alpha}$-benzoyl-L-phenylalanylthio)-2-D-methylpropanoyl]-L-proline-t-butyl ester.

The named product is synthesized by substituting the product from step 3 for the 3-mercapto-2-methylpropionic acid in Method XI, step 3 and substantially following the procedure described therein.

Step 5. Synthesis of $N^{\alpha}$-[3-($N^{\alpha}$-benzoyl-L-phenylalanylthio)-2-D-methylpropanoyl]-L-proline.

The named product is synthesized by removing the t-butyl ester group of the product of step 4 by following the procedure described in Method I, step 6(A)(ii).

XVII

This method involves the use of conventional solid phase technology for peptide synthesis. For a general reference to this technology, see Advan. Enzymol. 32, 221 (1969).

Step 1. Coupling L-Pro to a solid phase resin.

Using standard methodology, a solution of $N^{\alpha}$-Boc-L-Pro in ethyl acetate is mixed with a solid phase resin having 3-nitro-4-bromomethylbenzylamide groups attached thereto, in the presence of diisopropylethylamine. Excess $N^{\alpha}$-Boc-L-Pro is removed by washing with ethyl acetate, methanol and then dichloromethane. A solution of 25% TFA in dichloromethane containing 1 mg/ml of indole is then added to the resin having $N^{\alpha}$-Boc-L-Pro bound thereto resulting in the removal of the Boc protecting group. The resin is washed with dichloromethane.

Step 2. Coupling of 3-($N^{\alpha}$-benzoyl-L-phenylalanylthio)-2-D-methylpropionic acid to the product of Step 1.

A solution of the named compound and DCC in DMF and dichloromethane (1:1) mixed with the resin from step 1. The named compound is coupled to the proline in this step resulting in the product $N^{\alpha}$-[3-($N^{\alpha}$-benzoyl-L-phenylalanylthio)-2-D-methyl-propanoyl]-L-proline bound to the o-nitrobenzyl groups of the resin. The excess reactants are removed by washing with ethanol and then dichloromethane. The product is dried under a stream of dry nitrogen.

Step 3. Cleavage of $N^{\alpha}$-[3-($N^{\alpha}$-benzoyl-L-phenylalanylthio)-2-D-methylpropanoyl]-L-proline from the resin.

The material from step 2, in methanol, is irradiated

using light with a wavelength of 350 nm as described in J. Am. Chem. 97, 1575 (1975). The named product is washed from the resin using ethyl acetate. The solvent is removed by a rotary evaporator to yield the named product.

XVIII

Step 1. Coupling of L-Pro to a solid phase resin.

L-Pro is coupled to a resin as described in Method XXII, step 1.

Step 2. Coupling of 3-mercapto-2-methylpropionic acid to the product of step 1.

By substituting 3-Cbo-2-methylpropionic acid for the 3-($N^{\alpha}$-benzoyl-L-phenylalanylthio)-2-D-methylpropionic acid in Method XXII, step 2 and following the procedure of step 2, this compound is coupled to the L-Pro bound to the resin. A solution of TFA is mixed with the resin removing the Cbo group yielding the named compound. The resin is washed with ethanol and then dichloromethane.

Step 3. Coupling of L-Phe to the product of step 2.

A solution of $N^{\alpha}$-Boc - L-Phe and DCC in DMF and dichloromethane (1:1) is mixed with the resin of step 2. This product is coupled to the mercapto group of the compound attached to the resin. Excess reactants are removed by washing with ethanol and then dichloromethane. A solution of 25% TFA in dichloromethane (1 mg/ml indole) is mixed with the resin to remove the Boc protecting group resulting in $N^{\alpha}$-(3-L-phenylalanylthio-2-methylpropanoyl)-L-proline being bound to the resin. Excess TFA is removed by washing with dichloromethane.

Step 4. Coupling of the benzoyl group to the product of step 3.

A solution of benzoyl chloride in DMF containing 1 equivalent of TEA is mixed with the resin of step 3. The benzoyl group is coupled to the amino group of the Phe. The excess reactants are removed by washing with ethanol and the dichloromethane resulting in $N^{\alpha}$-[3-($N^{\alpha}$-benzoyl-L-phenylalanylthio)-2-methylpropanoyl]-L-proline bound to the resin.

Step 5. Cleavage of $N^{\alpha}$-[3-($N^{\alpha}$-benzoyl-L-phenyl alanylthio)-2-methylpropanoyl]-L-proline from the resin.

The named product is cleaved from the resin by following the procedure described in Method XXII, step 3.

Step 6. Resolution to yield $N^{\alpha}$-[3-(benzoyl-L-phenylalanylthio(-2-D-methylpropanoyl]-L-proline.

The named product is obtained by substantially following the procedure of Method X, step 2.

XIX

Step 1. Synthesis of L-thiophenylalanine hydrochloride.

L-thiophenylalanine phenyl ester hydrochloride is prepared by following the procedure described in Liebigs Ann. Chem. 576, 104 (1952).

The named product is prepared by substantially following the procedures described in Chem. Ber. 87, 1093 (1954) and Naturwissenschaften 40, 242 (1953).

Step 2. Synthesis of 3-(L-phenylalanylthio)-2-methylpropionic acid hydrochloride.

By substituting L-thiophenylalanine hydrochloride for the N-benzoyl-L-thiophenylalanine in Method I, step 4 and substantially following the procedure described therein, the named product is obtained.

Step 3. Resolution to yield 3-L-phenylalanylthio-2-D-methylpropionic acid.

The product from step 2 is resolved by substantially following the procedure described in Method I, step 5 or by ion exchange chromatography.

Step 4. Synthesis of 3-($N^{\alpha}$-benzoyl-L-phenylalanylthio)-2-D-methylpropionic acid.

10 mmoles of N-succinimidyl ester of benzoic acid is dissolved in cold THF and then this solution is added to a cold solution of 10 mmoles of the product from step 3 and 10 mmoles of $NaHCO_3$ in THF and water. The reaction mixture is stirred overnight at room temperature. The THF is removed with a rotary evaporator at 35°C. Water is added to the mixture and the pH is adjusted to using solid $NaHCO_3$. The aqueous phase is extracted with ethyl acetate. The aqueous phase is cooled in an ice bath and then acidified to pH 2 using 1N HCl in the presence of ethyl acetate. The organic phase is washed twice with cold water and then twice with saturated

NaCl. The organic phase is dried over anhydrous $MgSO_4$ and filtered. The solvent is removed with a rotary evaporator and the residue is crystallized from ether-hexane yielding the named product.

Step 5. Synthesis of $N^{\alpha}$-[3-(N-benzoyl-L-phenylalanylthio)-2-D-methyl]-L-proline.

The named product is synthesized by following the procedure described in Method I, step 6(A).

XX

Step 1. Synthesis of 3-($N^{\alpha}$-benzoyl-L-phenylalanylthio)-2-D-methylpropionic acid.

15 mmoles of 3-bromo-2-methylpropionic acid is dissolved in aqueous 1N NaOH and the solution is chilled in an ice bath. A mixture of 15 mmoles $N^{\alpha}$-benzoyl-L-thiophenylalanine and $K_2CO_3$ in water is added and the mixture is stirred overnight at room temperature. After acidification with concentrated HCl, the aqueous solution is extracted with ethyl acetate and the organic phase is washed with water, dried and concentrated to dryness. This product is then resolved by following the procedure described in Method I, step 5 to yield the named product. Alternatively, 3-bromo-2-D-methylpropionic acid can be used for the starting material thus eliminating the resolution step.

Step 2. Synthesis of $N^{\alpha}$-[3-($N^{\alpha}$-benzoyl-L-phenylalanylthio)-2-D-methylpropanoyl]-L-proline.

The named product is obtained by following the procedure described in Method I, step 6 (A).

XXI

Step 1. Synthesis of 3-benzyloxycarbonylthio-2-methylpropionic acid.

By substituting benzyl thioformate for the $N^{\alpha}$-benzoyl-L-thiophenylalanine in Method I, step 4 and substantially following the procedure described therein, the named product is obtained.

Step 2. Resolution to yield 3-benzyloxycarbonylthio-2-D-methylpropionic acid.

The named product is obtained by resolving the product of step 1 by crystallization as a salt or by liquid chromatography.

Step 3. Synthesis of $N^{\alpha}$-[3-(benzyloxycarbonylthio)-2-D-methylpropanoyl]-L-proline t-butyl ester.

By substituting the product of step 2 for the 3-($N^{\alpha}$-benzoyl-L-phenylalanylthio)-2-D-methylpropionic acid in Method I, step 6 (A)(i) and substantially following the procedure described therein, the named product is obtained.

Step 4. Synthesis of $N^{\alpha}$-(3-mercapto-2-D-methylpropanoyl)-L-proline.

The product from step 3 is suspended in anisole and reacted with HBr in acetic acid for 1/2 hour at room temperature with stirring. Ethyl ether is added to the mixture and cooled to 0°C for 1 hour. The precipitate is collected by filtration and washed several times with anhydrous ether. The desired product is dried in a vacuum desiccator over NaOH pellets and $P_2O_5$.

Step 5. Synthesis of $N^{\alpha}$-[3-$N^{\alpha}$-(benzoyl-L-phenylalanylthio)-2-D-methylpropanoyl]-L-proline.

The named product is obtained by substituting the product from step 4 for the 3-mercapto-2-methylpropionic acid in Method XI step 3 and substantially following the procedure described therein.

XXII

Step 1. Synthesis of 3-L-phenylalanylthio-2-methylpropionic acid.

Molar equivalents of $N^{\alpha}$-Boc-L-Phe and $H_2S$ are reacted using the mixed anhydride coupling method as described in J. Am. Chem. Soc. 74, 4726 (1952) to yield $N^{\alpha}$-Boc-L-thiophenylalanine. The Boc protecting group is removed by following the procedure described in Method I, step 2 (i). In this procedure the TFA and anisole are completely removed before proceeding. By substituting the resulting L-thiophenylalanine for the N-benzoyl-L-thiophenylalanine of Method I, step 4 and substantially following the procedure described therein, the named product is obtained.

Step 2. Synthesis of 3-($N^{\alpha}$-benzoyl-L-phenylalanylthio)-2-methylpropionic acid.

2 mmoles of the product from step 1 is suspended in THF and water (7:3) containing 4 mmoles of $NaHCO_3$ with vigorous stirring. Then 2 mmoles of benzoyl chloride at room

0038117

temperature is added and the resulting mixture stirred vigorously for 1 hour. Ethyl acete is added and the mixture is adjusted to pH 2 with 1 N HCl at 0°C. The organic phase is washed with saturated NaCl, dried over $MgSO_4$ and filtered. The solvent is removed with a rotary evaporator. The residue is crystallized and recrystallized from benzene n-hexane yielding white crystals.

Step 3. Resolution to yield 3-($N^{\alpha}$-benzoyl-L-phenylalanylthio)-2-D-methylpropionic acid.

The named product is obtained by following the procedure described in Method I, step 5.

Step 4. Synthesis of $N^{\alpha}$-[3-($N^{\alpha}$-benzoyl-L-phenylalanylthio)-2-D-methylpropanoyl]-L-proline.

The named product is obtained by following the procedure described in Method I, step 6(A).

XXIII

Step 1. Synthesis of 3-acetylthio-2-methylpropionic acid o-nitrobenzyl ester.

10 mmoles of 3-acetylthio-2-methylpropionic acid in 2-fluoro-1,3,5-trinitrobenzene (FTNB) is added to a solution of 10 mmoles of o-nitrobenzyl alcohol in FTNB with stirring at room temperature. The reaction mixture is stirred for 1 1/2 hours and the solvent is then removed by a rotary evaporator yielding the named compound.

Step 2. Resolution to yield 3-acetylthio-2-D-methylpropionic acid o-nitrobenzyl ester.

The product from step 1 is resolved using liquid chromatography to yield the named product.

Step 3. Synthesis of 3-acetylthio-2-D-methylpropionic acid.

The product from step 2 is irradiated using light with a wavelength of 350 nm as described in J. Am. Chem. Soc. 97, 1575 (1975).

Step 4. Synthesis of $N^{\alpha}$-(3-acetylthio-2-D-methylpropanoyl)-L-proline t-butyl ester.

By substituting the product from step 3 for the 3-($N^{\alpha}$-benzoyl-L-phenylalanylthio)-2-D-methylpropionic acid in Method I, step 6 (A)(i) and substantially following the procedure described therein, the named product is obtained.

Step 5. Synthesis of $N^{\alpha}$-(3-mercapto-2-D-methylpropanoyl)-L-proline t-butyl ester.

The product from step 4 is dissolved in a mixture of concentrated ammonia, methanol and anisole. After 1 hour the solvent is removed under pressure and the residue is crystallized from benzene-hexane to yield the named product.

Step 6. Synthesis of $N^{\alpha}$-[3-($N^{\alpha}$-benzoyl-L-phenylalanylthio)-2-D-methylpropanoyl]-L-proline t-butyl ester.

By substituting the product from step 5 for the 3-mercapto-2-D-nethylpropanoyl-L-proline in Method XI step 3 and following the procedure described therein, the named product is obtained.

Step 7. Synthesis of $N^{\alpha}$-[3-($N^{\alpha}$-benzoyl-L-phenylalanylthio)-2-D-methylpropanoyl]-L-proline.

The t-butyl ester is removed from the product of step 6 by following the procedure described in Method I, step 6 (A) (ii) to yield the named product.

XXIV

Step 1. Resolution to yield 3-amino-2-D-methylpropionic acid.

3-amino-2-methylpropionic acid is resolved to yield the named compound by following the procedure described in J. Biol. Chem. 236, 3283 (1961).

Step 2. Synthesis of 3-bromo-2-D-methylpropionic acid.

The product from step 1 is dissolved in water and the resulting solution is cooled to -5°C. To this solution is added a little $NaNO_2$ and 48% HBr in water. The mixture is stirred for 30 minutes and then slowly warmed to room temperature. The solvent is removed by a rotary evaporator yielding the named product.

Step 3. Synthesis of 3-($N^{\alpha}$-benzoyl-L-phenylalanylthio)-2-D-methylpropionic acid.

The named product is obtained by following the procedure described in Method XXV, step 1.

Step 4. Synthesis of $N^{\alpha}$-[3-($N^{\alpha}$-benzoyl-L-phenylalanylthio)-2-D-methylpropanoyl]-L-proline.

The named product is obtained by following the procedure described in Method I, step 6(A).

XXV

Step 1. Resolution to yield 3-amino-2-D-methylpropionic acid.

3-amino-2-methylpropionic acid is resolved to yield the named compound following the procedure described in J. Biol. Chem. 236, 3283 (1961).

Step 2. Synthesis of 3-bromo-2-D-methylpropionic acid.

The named product is obtained by following the procedure described in Method XXIX, step 2.

Step 3. Synthesis of 3-benzoylthio-2-D-methylpropionic acid.

By substituting thiobenzoic acid for the N-benzoyl-L-thiophenylalanine in Method XXV step 1 and using the product from step 2 and following the procedure described therein, the named product is obtained.

Step 4. Synthesis of 3-mercapto-2-D-methylpropionic acid.

The benzoyl group is removed by following the procedure described in Method XI step 2 to yield the named product.

Step 5. Synthesis of 3-($N^{\alpha}$-benzoyl-L-phenylalanylthio)-2-D-methylpropionic acid.

The named product is prepared by following the procedure described in Method XI, step 3 utilizing the product from step 4 as one of the reactants.

Step 6. Synthesis of $N^{\alpha}$-[3-($N^{\alpha}$-benzoyl-L-phenylalanylthio)-2-D-methylpropanoyl]-L-proline.

The named product is synthesized by following the procedure described in Method XI step 5.

XXVI

Step 1. Synthesis of 3-benzoylthio-2-D-methylpropionic acid t-butyl ester.

25 mmoles of 3-benzoylthio-2-D-methylpropionic acid is dissolved in dichloromethane plus 0.5 ml concentrated sulfuric acid. A stream of isobutylene is passed through the solution with stirring for 60 minutes. The reaction vessel is closed and the solution stirred for 16 hours. The named product is extracted from water with ether. The solvent is removed with a rotary evaporator and then high vacuum.

Step 2. Synthesis of 3-mercapto-2-D-methylpropionic acid t-butyl ester.

The product from step 1 is dissolved in methanolic ammonia under nitrogen gas. After one hour, the solvent is removed. The residue is dissolved in ether, cooled in an ice bath and then washed with 0.1N HCl and then saturated NaCl. The organic phase is dried over anhydrous $MgSO_4$ and then filtered. Solvent is removed with a rotary evaporator. The named product is obtained by vacuum distillation.

Step 3. Synthesis of 3-($N^\alpha$-benzoyl-L-phenylalanylthio)-2-D-methylpropionic acid t-butyl ester.

(A) (i) Preparation of active ester. A solution of 25 mmoles of $N^\alpha$-benzoyl-L-Phe in redistilled ethyl acetate is cooled to -20°C in a methanol-ice-dry ice bath. The $N^\alpha$-benzoyl-L-Phe is prepared as described in Serial No. 128,953, e.g. by following the procedure of Carter et al., J. Biol. Chem. 138, 627 (1941). To this solution is added 25 mmoles of N-ethyl morpholine followed by 25 mmoles of isobutylchloroformate and the solution is stirred for several minutes at -15°C. 50 mmoles of N-hydroxysuccinimide is added and the resulting reaction mixture stirred for 1 1/2 hours at -15°C. (ii) Active ester method. The reaction mixture is slowly warmed to room temperature and 25 mmoles of 3-mercapto-2-D-methylpropionic acid t-butyl ester (from step 2) is added and then stirred until the reaction, as judged by thin layer chromatography, is completed. Ethyl acetate is added and the mixture cooled in an ice bath and acidified to pH 3 with 1 N HCl. The organic phase is washed several times with cold water and cold saturated NaCl. The organic phase is then dried over anhydrous $MgSO_4$ and filtered. The solvent is removed with a rotary evaporator. The residue is crystallized from benzene-petroleum ether yielding the named product.

(B) A solution of 25 mmoles of $N^\alpha$-benzoyl-L-Phe in tetrahydrofuran is added with stirring to 25.1 mmoles of EDDQ. 25 mmoles of 3-mercapto-2-D-methylpropionic acid t-butyl ester is added. The resulting mixture is then stirred until

the reaction is completed, as judged by thin layer chromatography. The solvent is removed with a rotary evaporator. The product is crystallized from benzene-petroleum ether.

Step 4. Synthesis of 3-($N^{\alpha}$-benzoyl-L-phenylalanylthio)-2-D-methylpropionic acid.

The product from step 3 is suspended in 4 ml of anisole and reacted with 9 ml of TFA for 1 1/2 hours at room temperature with stirring. TFA and anisole is removed with a rotary evaporator and then under high vacuum over NaOH and $P_2O_5$ to yield the named product.

Step 5. Synthesis of $N^{\alpha}$-[3-($N^{\alpha}$-benzoyl-L-phenylalanylthio)-2-D-methylpropanoyl]-L-proline.

(A) A cool solution of 25 mmoles of DCC in dimethylformamide (DMF) is added drop-wise to a mixture of 25 mmoles of the product from step 4 and 25 mmoles of N-hydroxysuccinimide in DMF at 0°C. The reaction mixture is stirred for 30 minutes at 0°C and then overnight at 4°C. Crystalline dicyclohexylurea is removed by filtration and the precipitate washed with ethyl acetate. Solvents from the combined filtrates are removed under reduced pressure and the residue crystallized from benzene-hexane. The crystals are dissolved in cold THF and then this solution is added to a cold solution of 25 mmoles of L-Pro and 25 mmoles of $NaHCO_3$ in THF/water. The reaction mixture is stirred overnight at room temperature. The THF is removed with a rotary evaporator at 35°C. Water is added to the mixture and the pH is adjusted to 9 using solid $NaHCO_3$. The aqueous phase is extracted with ethyl acetate. The aqueous phase is cooled in an ice bath and then acidified to pH 2 using 1 N HC1 in the presence of ethyl acetate. The organic phase is washed twice with cold water and then twice with saturated NaC1. The organic phase is dried over anhydrous $MgSO_4$ and filtered. The solvent is removed with a rotary evaporator and the residue is crystallized from ether-hexane yielding the named product.

(B) (i) 25 mmoles of the product from step 4 is dissolved in ethyl acetate and cooled to 0°C in an ice-acetone bath. 25 mmoles of DCC in ethyl acetate is added and the mixture stirred for 5 minutes at 0°C. To this solution is added 25 mmoles of L-proline t-butyl ester in

ethyl acetate. The reaction mixture is stirred overnight at 4°C. The reaction mixture is worked-up by addition of 10 ml of ethyl acetate and filtration. The precipitate is washed with 20 ml of ethyl acetate. The combined ethyl acetate fractions are chilled in a freezer. The organic phase is washed three times with saturated NaCl, once with cold 0.1 N $NaHCO_3$ and three times with saturated NaCl. The organic phase is dried with anhydrous $MgSO_4$ and filtered. Solvent is removed with a rotary evaporator under high vacuum yielding a white residue. (ii) The residue is suspended in 4 ml of anisole and reacted with 8 ml of TFA for 1 1/2 hours at room temperature with stirring. TFA is removed with a rotary evaporator under high vacuum. The residue is dissolved in 1 ml of tetrahydrofuran (THF) and chromatographed. The desired fractions are evaporated to dryness, dissolved in a small amount of isopropanol and rechromatographed. The desired fractions yielding an oil are dissolved in THF and then the THF is evaporated under a stream of $N_2$. The product is obtained after removing the remaining solvent with a rotary evaporator under high vacuum.

XXVII

Step 1. Synthesis of 3-($N^{\alpha}$-Bpoc-L-phenylalanylthio)-2-D-methylpropionic acid t-butyl ester.

$N^{\alpha}$-Bpoc-L-Phe is synthesized by following the procedure described by Sieber et al., Helv. Chem. Acta 51, 614 (1968). 3-mercapto-2-D-methylpropionic acid t-butyl ester is prepared as described in Method II steps 1 and 1. The named product is prepared by substituting $N^{\alpha}$-Bpoc-L-Phe for the $N^{\alpha}$-Bz-L-Phe in Method II step 3(A) and substantially following the procedure described therein.

Step 2. Synthesis of the trifluoroacetate salt of 3-(L-phenylalanylthio)-2-D-methylpropionic acid t-butyl ester.

The product from step 1 is dissolved in methylene chloride containing 0.5% TFA and stirred for 10 minutes. The methylene chloride and excess TFA are removed with a rotary evaporator to yield the named product.

Step 3. Synthesis of 3-($N^{\alpha}$-benzoyl-L-phenylalanylthio)-2-D-methylpropionic acid t-butyl ester.

15 mmoles of the product from step 2 is suspended in ethyl acetate with vigorous stirring. Then 15 mmoles of benzoyl chloride at room temperature is added. To this mixture is added drop-wise a solution of 32 mmoles of $NaHCO_3$ in water and the resulting mixture stirred vigorously for another 30 minutes. The mixture is then cooled in an ice bath for another 30 minutes. Ethyl acetate is added to the mixture. The mixture is cooled in an ice bath and washed with water, with 1 N citric acid and then several times with saturated NaCl. The solvent is removed with a rotary evaporator after drying over anhydrous $MgSO_4$ to yield the named product.

Step 4. Synthesis of 3-($N^{\alpha}$-benzoyl-L-phenylalanylthio)-2-D-methylpropionic acid.

The t-butyl group is removed from the product of step 3 by following the procedure described in Method II step 4 to yield the named product.

Step 5. Synthesis of $N^{\alpha}$-[3-($N^{\alpha}$-benzoyl-L-phenylalanylthio)-2-D-methylpropanoyl]-L-proline.

The named product is prepared by following the procedure described in Method II step 5.

XXVIII

Step 1. Synthesis of 3-mercapto-2-D-methylpropionic acid.

The named product is obtained by following the procedure described in Method II step 2 and substituting 3-benzoylthio-2-D-methylpropionic acid for its t-butyl ester therein.

Step 2. Synthesis of 3-($N^{\alpha}$-Boc-L-phenylalanylthio)-2-D-methylpropionic acid.

By substituting $N^{\alpha}$-Boc-L-Phe for the $N^{\alpha}$-Bz-L-Phe and the product from step 1 for the 3-mercapto-2-D-methylpropionic acid t-butyl ester in Method II, step 3(A) and substantially following the procedure described therein, the named product is obtained.

Step 3. Synthesis of the trifluoroacetate salt of 3-L-phenylalanylthio-2-D-methylpropionic acid.

The product from step 2 is suspended in 4 ml of anisole and reacted with 8 ml of TFA for 1 1/2 hours at room temperature with stirring. The anisole and excess TFA are removed with a rotary evaporator to yield the named product.

Step 4. Synthesis of 3-($N^{\alpha}$-benzoyl-L-phenylalanylthio)-2-D-methylpropionic acid.

By substituting the product from step 3 for the TFA salt of 3-L-phenylalanylthio-2-D-methylpropionic acid t-butyl ester in Method III, step 3 and substantially following the procedure described therein, the named product is obtained.

Step 5. Synthesis of $N^{\alpha}$-[3-($N^{\alpha}$-benzoyl-L-phenylalanylthio)-2-D-methylpropanoyl]-L-proline.

The named product is obtained by following the procedure described in Method II step 5.

XXIX

Step 1. Synthesis of 3-($N^{\alpha}$-Bpoc-L-phenylalanylthio)-2-D-methylpropionic acid.

The named compound is prepared by following the procedure described in Method III step 1 and substituting 3-mercapto-2-D-methylpropionic acid (prepared as described in Method V step 1) for its t-butyl ester therein.

Step 2. Synthesis of $N^{\alpha}$-[3-($N^{\alpha}$-Bpoc-L-phenylalanylthio)-2-D-methylpropanoyl]-L-proline t-butyl ester.

A cool solution of 10 mmoles of DCC in DMF and also containing 10 mmoles of 1-hydroxybenzotriazole is added dropwise to a mixture of 10 mmoles of the product from step 1 and 10 mmoles of L-proline t-butyl ester in DMF. The reaction mixture is stirred for 30 minutes at 0°C and then overnight at 4°C. Crystalline dicyclohexylurea is removed by filtration and the precipitate is washed with ethyl acetate. Solvents from the combined filtrates are removed under pressure and the residue is crystallized from ether-hexane to yield the named product.

Step 3. Synthesis of the trifluoroacetate salt of $N^{\alpha}$-[3-L-phenylalanylthio-2-D-methylpropanoyl]-L-proline t-butyl ester.

The named product is obtained by following the procedure described in Method III step 2 and substituting the product of step 1 for the 3-($N^{\alpha}$-Bpoc-L-phenylalanylthio)-2-D-methyl-

propionic acid t-butyl ester therein.

Step 4. Synthesis of $N^{\alpha}$-[3-($N^{\alpha}$-benzoyl-L-phenylalanylthio)-2-D-methyl-propanoyl]-L-proline t-butyl ester.

By substituting the product from step 3 for the TFA salt of 3-L-phenylalanylthio-2-D-methylpropionic acid t-butyl ester in Method III, step 3 and substantially following the procedure described therein, the named product is prepared.

Step 5. Synthesis of $N^{\alpha}$-[3-$N^{\alpha}$(-benzoyl-L-phenylalanyl)-2-D-methyl-propanoyl]-L-proline.

The named product is prepared by following the procedure described in Method II, step 5(B)(ii).

XXX

Step 1. Synthesis of $N^{\alpha}$-[3-($N^{\alpha}$-Boc-L-phenyl-alanylthio)-2-D-methylpropanoyl]-L-proline.

3-($N^{\alpha}$-Boc-L-phenylalanylthio)-2-D-methylpropionic acid is prepared as described in Method V, step 2. This product is substituted for the 3-($N^{\alpha}$-benzoyl-L-phenylalanylthio)-2-D-methylpropionic acid in Method II step 5(A) and the procedure described therein is substantially followed to produce the named compound.

Step 2. Synthesis of the trifluoroacetate salt of $N^{\alpha}$-[3-L-phenylalanylthio-2-D-methyl-propanoyl]-L-proline.

The named product is prepared by following the procedure described in Method V, step 3.

Step 3. Synthesis of $N^{\alpha}$-[3-($N^{\alpha}$-benzoyl-L-phenylalanylthio)-2-D-methylpropanoyl]-L-proline.

By substituting the product from step 2 for the TFA salt of 3-L-phenylalanylthio-2-D-methylpropionic acid t-butyl ester in Method III, step 3 and substantially following the procedure described therein, the named product is obtained.

While the invention has been described in connection with specific embodiments thereof, it will be understood that it is capable of further modifications and this application is intended to cover any variations, uses, or adaptations of the invention following, in general, the principles of the invention and including such departures from the present

disclosure as come within known or customary practice within the art to which the invention pertains and as may be applied to the essential features hereinbefore set forth, and as follows in the scope of the appended claims.

CLAIMS

1. A method for preparing a compound having the formula:

$$R - A - S - (CH_2)_n - \underset{\displaystyle R_1}{\underset{|}{CH}} - \overset{\displaystyle O}{\overset{\|}{C}} - R_2$$

wherein

R is hydrogen, t-butyloxycarbonyl, cyclopentanecarbonyl-L-lysyl, pyro-L-glutamyl-L-lysyl, L-lysyl, L-arginyl, pyro-L-glutamyl or R', where R' is formyl, acetyl, propanoyl, butanoyl, phenylacetyl, phenylpropanoyl benzoyl or cyclopentanecarbonyl;

A is phenylalanyl, alanyl, tryptophyl, tyrosyl, glycyl, isoleucyl, leucyl, histidyl, or valyl, the α-amino group thereof being in amide linkage with R:

$R_1$ is hydrogen or methyl;

$R_2$ is L-proline, L-3,4-dehydroproline, D,L-3,4-dehydroproline, L-3-hydroxyproline, L-4-hydroxyproline, L-thiazolidine-4-carboxylic acid, or L-5-oxo-proline, the imino group thereof being imide linkage with the adjacent $-\overset{O}{\overset{\|}{C}}-$; and

$R_3$ is an acid sensitive amino protecting group;

$R_4$ is a photosensitive amino protecting group;

n is 0 or 1, such that when n is 0, $R_1$ is methyl; and where n=1 and $R_1$ is methyl, the $-CH_2-\underset{\displaystyle CH_3}{\underset{|}{CH}}-$ moiety is in the D-configuration, selected from the group consisting of:

I. a method comprising the steps of

(A) reacting a compound having the formula R-A with an aryl thiol or alkyl thiol in the presence of a coupling agent, with the proviso that when R is R' and A is an amino acid in either the D- or L- form in the compound prepared by this process, R is $R_3$ in the compound R-A and step (B) is performed,

(B) removing the $R_3$ protecting group from the product

of step (A) and reacting the resulting A-S aryl or alkyl ester with an R' coupling compound,

(C) reacting the product of step (A) or (B) with alkali metal hydrogen sulfide or $H_2S$ to produce a compound having the formula R-A-SH,

(D) reacting the product of step (C) with acrylic acid or methacrylic acid to produce a compound having the formula $R-A-S-CH_2-\underset{R_1}{CH}-\overset{O}{\overset{\|}{C}}-OH$, wherein $R_1$ is hydrogen or methyl, respectively, with the proviso that when $R_1$ is methyl, step (E) is performed,

(E) resolving the product of step (D) to produce a compound in which $-CH_2-\underset{R_1}{\underset{|}{CH}}-$ is in the D- configuration,

(F) coupling the product of step (D) or (E) with $R_2$;

II. a method comprising the steps of

(A) reacting a compound having the formula R-A with an arylthiol or alkylthiol in the presence of a coupling agent,

(B) reacting the product of step (A) with alkali metal hydrogen sulfide or $H_2S$ to produce a compound having the formula R-A-SH;

(C) reacting the product of step (B) with acrylic acid or methacrylic acid to produce a compound having the formula $R-A-S-CH_2-\underset{R_1}{CH}-\overset{O}{\overset{\|}{C}}-OH$, wherein $R_1$ is hydrogen or methyl, respectively, with the proviso that when $R_1$ is hydrogen, step (D) is not performed,

(D) resolving the product of step (C) to produce a compound in which $-CH_2-\underset{R_1}{CH}-$ is in the D-configuration,

(E) coupling the product of step (C) or (D) with $R_2$,

(F) removing the $R_3$ protecting group and reacting the resulting product with an R' coupling compound to produce a compound having the formula $R'-A-S-CH_2-\underset{R_1}{CH}-\overset{O}{\overset{\|}{C}}-OH$, when R is R' and A is an amino acid in the D- or L-form in the compound prepared by this process and R is $R_3$ in compound R-A;

III. a method comprising the steps of

(A) reacting a compound having the formula R-A with pentachlorophenol, pentafluorophenol or N-hydroxysuccinimide, with the proviso that when R is R' and A is an amino acid in the D- or L- form in the compound prepared by this process, R is $R_3$ in compound R-A and steps (B) and (C) are performed,

(B) removing the $R_3$ protecting group from the product of step (A),

(C) reacting the product from step (B) with an R' coupling compound,

(D) reacting the product from step (A) or (C) with a compound having the formula $HS-(CH_2)_n-CH(R_1)-C(=O)-OH$, with the proviso that when n is 1 and $R_1$ is methyl, step (E) is performed,

(E) resolving the product of step (D) to produce a compound in which $-(CH_2)_n-CH(R_1)-$ is in the D-configuration,

(F) coupling the product of step (D) or (E) with $R_2$;

IV. a method comprising the steps of

(A) reacting a compound having the formula R-A with $H_2S$ to produce a compound having the formula R-A-SH,

(B) reacting the product of step (A) with acrylic acid or methacrylic acid to produce a compound having the formula $R-A-S-CH_2-CH(R_1)-C(=O)-OH$, wherein $R_1$ is hydrogen or methyl, respectively, with the proviso that when $R_1$ is methyl, step (C) is performed,

(C) resolving the product of step (B) to produce a compound in which $-CH_2-CH(R_1)-$ is in the D- configuration,

(D) coupling the product of step (B) or (C) with $R_2$;

V. a method comprising the steps of

(A) coupling $R_2$ to acrylic acid, methacrylic acid, or an active derivative thereof, to produce a compound having the formula $H_2C=C(R_1)-C(=O)-R_2$, wherein $R_1$ is hydrogen or methyl, respectively,

(B) reacting the product of step (A) with R-A-SH, with the proviso that when $R_1$ is methyl step (C) is performed,

(C) resolving the product of step (B) to produce a compound in which $-CH_2-\underset{R_1}{CH}-$ is in the D- configuration,

VI. a method comprising the steps of

(A) reacting A with an R coupling compound to produce a compound having the formula R-A,

(B) reacting thioacetic acid with acrylic acid or methacrylic acid and then removing the acetyl group from the resulting product,

(C) reacting the product from step (A) with the product of step (B) to produce a compound having the formula

$R-A-S-CH_2-\underset{R_1}{CH}-\overset{O}{\overset{\|}{C}}-OH$, wherein $R_1$ is hydrogen or methyl, with the proviso that when $R_1$ is methyl step (D) is performed,

(D) resolving the product of step (C) to produce a compound which $-CH_2-\underset{R_1}{CH}-$ is in the D- configuration,

(E) coupling the product of step (C) or (D) with $R_2$;

VII. a method comprising the steps of

(A) reacting a compound having the formula R-A with an arylthio or alkylthiol in the presence of a coupling agent, with the proviso that when R is R' and A is an amino acid in the D- or L-form in the compound prepared by this process, R is $R_3$ in compound R-A, $R_3$ is removed after reacting said thiol with R-A, and the resulting product is reacted with an R' coupling compound,

(B) reacting the product of step (A) with alkali metal hydrogen sulfide or $H_2S$ to produce a compound having the formula R-A-SH,

(C) reacting the product of step (B) with acrylic acid or methacrylic acid to produce a compound having the formula

$R-A-S-CH_2-\underset{R_1}{CH}-\overset{O}{\overset{\|}{C}}-OH$, wherein $R_1$ is hydrogen or methyl, respectively, with the proviso that when $R_1$ is methyl, step (D) is performed,

(D) resolving the product of step (C) to produce a compound in which $-CH_2-\underset{R_1}{CH}-$ is in the D- configuration,

(E) coupling the product of step (C) or (D) with $R_2$;

VIII. a method comprising the steps of

(A) reacting a compound having the formula R-A with an arylthiol or alkylthiol in the presence of a coupling agent,

(B) reacting the product of step (A) with alkali metal hydrogen sulfide or $H_2S$ to produce a compound having the formula R-A-SH,

(C) reacting the product of step (B) with acrylic acid or methacrylic acid to produce a compound having the formula $R-A-S-CH_2-\underset{R_1}{CH}-\overset{O}{\overset{\|}{C}}-OH$ wherein $R_1$ is hydrogen or methyl, respectively, with the proviso that when $R_1$ is methyl step (D) is performed,

(D) resolving the product of step (C) to produce a compound in which $-CH_2-\underset{R_1}{CH}-$ is in the D-configuration,

(E) removing the $R_3$ protecting group and reacting the resulting product with a benzoyl coupling compound to produce a compound having the formula $R'-A-S-CH_2-\overset{R_1}{\overset{|}{CH}}-\overset{O}{\overset{\|}{C}}-OH$, when R is R' and A is an amino acid in the D- or L- form in the compound prepared by this process and R is $R_3$ in compound R-A,

(F) coupling the product of step (C), (D) or (E) with $R_2$;

IX. a method comprising the steps of

(A) reacting A with an R coupling compound to produce a compound having the formula R-A,

(B) reacting the product of step (A) with a compound having the formula $HS-(CH_2)_n-\overset{R_1}{\overset{|}{CH}}-\overset{O}{\overset{\|}{C}}-OH$, with the proviso that when n is 1 and $R_1$ is methyl step (C) is performed,

(C) resolving the product of step (B) to produce a compound in which $-(CH_2)_n-\overset{R_1}{\overset{|}{CH}}-$ is in the D- configuration,

(D) coupling the product of step (B) or (C) with $R_2$,

X. a method comprising the steps of

(A) reacting a compound having the formula $R_4$-A with a compound having the formula $HS-(CH_2)_n-\overset{R_1}{\overset{|}{C}}H-\overset{O}{\overset{\|}{C}}-OH$, with the proviso that when n is 1 and $R_1$ is methyl, step (B) is performed,

(B) resolving the product of step (A) to produce a compound in which $-(CH_2)_n-\overset{R_1}{\overset{|}{C}}H-$ is in the D- configuration,

(C) coupling the product of step (A) or (B) with $R_2$,

(D) removing the $R_4$ protecting group from the product of step (C) by irradiation,

(E) reacting the product of step (D) with an R coupling compound,

XI. a method comprising the steps of

(A) reacting a compound having the formula $R_3$-A with $HS-(CH_2)_n-\overset{R_1}{\overset{|}{C}}H-\overset{O}{\overset{\|}{C}}-OH$, with the proviso that when n is 1 and $R_1$ is methyl, step (B) is performed,

(B) resolving the product of step (A) to produce a compound in which $-(CH_2)_n-\overset{R_1}{\overset{|}{C}}H-$ is in the D- configuration,

(C) coupling the product of step (A) or (B) with $R_2$,

(D) removing the $R_3$ protecting group from the product of step (C),

(E) reacting the product of step (D) with an R coupling compound;

XII. a method comprising the steps of

(A) reacting a compound having the formula $H_3C-\overset{O}{\overset{\|}{C}}-S-(CH_2)_n-\overset{R_1}{\overset{|}{C}}H-\overset{O}{\overset{\|}{C}}-OH$ with 2-methylpropene and then removing the acetyl group from the resulting product,

(B) reacting the product of step (A) with a compound having the formula R-A to produce a compound having the formula $R-A-S-(CH_2)_n-\overset{R_1}{\overset{|}{C}}H-\overset{O}{\overset{\|}{C}}-OC(CH_3)_3$,

(C) removing the t-butyl group from the product of step (B), with the proviso that when n is 1 and $R_1$ is methyl step (D) is performed,

(D) resolving the product of step (C) to produce a

compound in which $-(CH_2)_n-\overset{R_1}{\overset{|}{C}}H-$ is in the D- configuration,

(E) coupling the product of step (C) or (D) with $R_2$,

XIII. a method comprising the steps of

(A) reacting a compound having the formula $H_3C-\overset{O}{\overset{\|}{C}}-S-(CH_2)_n-\overset{R_1}{\overset{|}{C}}H-\overset{O}{\overset{\|}{C}}-OH$ with 2-methylpropene and then removing the acetyl group from the resulting product,

(B) reacting the product of step (A) with a compound having the formula R-A to produce a compound having the formula $R-A-S-(CH_2)_n-\overset{R_1}{\overset{|}{C}}H-\overset{O}{\overset{\|}{C}}-O-C(CH_3)_3$ wherein R is $R_3$ or $R_4$,

(C) removing the $R_3$ or $R_4$ protecting group from the product of step (B),

(D) reacting the product of step (C) with an R coupling compound,

(E) removing the t-butyl group from the product of step (D), with the proviso that when n is 1 and $R_1$ is methyl step (F) is performed,

(F) resolving the product of step (E) to produce a compound in which $-(CH_2)_n-\overset{R_1}{\overset{|}{C}}H-$ is in the D- configuration,

(G) coupling the product of step (E) or (F) with $R_2$,

XIV. a method comprising the steps of

(A) reacting a compound having the formula R-A, wherein R is $R_3$ or $R_4$, with a compound selected from the group consisting of N-hydroxysuccinimide, p-nitrophenol, o-nitrophenol, N-hydroxybenzotriazole, pentachlorophenol, pentafluorophenol and N-hydroxy-5-norbenen-2,3-dicarboximide, and the resulting product is reacted with alkali metal hydrogen sulfide or $H_2S$ to produce a compound having the formula R-A-SH,

(B) reacting the product of step (A) with acrylic acid or methacrylic acid to produce a compound having the formula $R-A-S-CH_2-\overset{R_1}{\overset{|}{C}}H-\overset{O}{\overset{\|}{C}}-OH$ wherein $R_1$ is hydrogen or methyl, respectively, with the proviso that when n is 1 and $R_1$ is methyl, step (C) is performed,

(C) resolving the product of step (B) to produce a

compound in which $-CH_2-\overset{R_1}{\overset{|}{C}}H-$ is in the D- configuration,

(D) coupling the product of step (B) or (C) with $R_2$,

(E) removing the $R_3$ or $R_4$ protecting group from the product of step (D),

(F) reacting the product of step (E) with an R coupling compound;

XV. a method comprising the steps of

(A) coupling $R_2$ to acrylic acid or methacrylic acid to produce a compound having the formula $H_2C=\overset{R_1}{\overset{|}{C}}-\overset{O}{\overset{\|}{C}}-R_2$,

(B) reacting a compound having the formula R-A, wherein R is $R_3$ or $R_4$, with a compound selected from the group consisting of N-hydroxysuccinimide, p-nitrophenol, o-nitrophenol, N-hydroxybenzotriazole, N-hydroxy-5-norbornene-2,3-dicarboximide, pentachlorophenol or pentafluorophenol, and the resulting product is reacted with alkali metal hydrogen sulfide or $H_2S$ to produce a compound having the formula R-A-SH,

(C) reacting the product of step (B) with the product of step (B)

(D) removing the $R_3$ or $R_4$ protecting group from the product of step (C),

(E) reacting the product of step (D) with an R coupling compound to produce a compound having the formula

$R-A-S-CH_2-\overset{R_1}{\overset{|}{C}}H-\overset{O}{\overset{\|}{C}}-R_2$ wherein R is hydrogen or methyl with the proviso that when $R_1$ is methyl, step (F) is performed,

(F) resolving the product of step (E) to produce a compound in which $-CH_2-\overset{R_1}{\overset{|}{C}}H-$ is in the D- configuration;

XVI. a method comprising the steps of

(A) coupling $R_2$ to a compound having the formula

$H_3C-\overset{O}{\overset{\|}{C}}-S-(CH_2)_n-\overset{R_1}{\overset{|}{C}}H-\overset{O}{\overset{\|}{C}}-OH$,

(B) removing the acetyl group from the product of step (A) to produce a compound having the formula

$HS-(CH_2)_n-\overset{R_1}{\overset{|}{C}}H-\overset{O}{\overset{\|}{C}}-R_2$ wherein $R_1$ is hydrogen or methyl, with the proviso that when n is 1 and $R_1$ is methyl, step (C) is performed,

(C) resolving the product of step (B) to produce a compound in which $-(CH_2)_n-\overset{R_1}{\overset{|}{C}}H-$ is in the D- configuration,

(D) reacting the product of step (B) or (C) with a compound having the formula R-A;

XVII. a method comprising the steps of

(A) coupling a compound having the formula $R_3-R_2$ to a solid phase resin capable of reacting with $R_2$, and removing the $R_3$ protecting group from the resulting complex,

(B) coupling a compound having the formula

$R-A-S-(CH_2)_n-\overset{R_1}{\overset{|}{C}}H-\overset{O}{\overset{\|}{C}}-OH$ to the $R_2$-resin complex of step (A),

(C) cleaving the resulting complex of step (B) to produce a compound having the formula $R-A-S-(CH_2)_n-\overset{R_1}{\overset{|}{C}}H-\overset{O}{\overset{\|}{C}}-R_2$, wherein $R_1$ is hydrogen or methyl, with the proviso that when n is 1 and $R_1$ is methyl, step (D) is performed,

(D) resolving the product of step (C) to produce a compound in which $-(CH_2)_n-\overset{R_1}{\overset{|}{C}}H-$ is in the D- configuration;

XVIII. a method comprising the steps of

(A) coupling a compound having the formula $R_3-R_2$ to a solid phase resin capable of reacting with $R_3$, and removing the $R_3$ protecting group from the resulting complex,

(B) coupling a compound having the formula

$Cbo-S-(CH_2)_n-\overset{R_1}{\overset{|}{C}}H-\overset{O}{\overset{\|}{C}}-OH$ to the $R_2$-resin complex of step (A) and removing the Cbo group from the resulting complex,

(C) coupling a compound having the formula $R_3-A$ to the complex of step (B),

(D) removing the $R_3$ protecting group from the complex of step (C),

(E) coupling an R coupling compound to the complex of step (D),

(F) cleaving the complex of step (E) to produce a compound having the formula $R-A-S-(CH_2)_n-\overset{R_1}{\overset{|}{C}}H-\overset{O}{\overset{\|}{C}}-R_2$, wherein $R_1$ is hydrogen or methyl, with the proviso that when n is 1 and $R_1$

is methyl, step (G) is performed,

(G) resolving the product of step (F) to produce a compound in which $-(CH_2)_n-\overset{R_1}{\overset{|}{C}}H-$ is in D- configuration;

XIX. a method comprising the steps of

(A) reacting a compound having the formula A-thio ester with $H_2S$ to produce a compound having the formula A-SH,

(B) reacting the product of step (A) with acrylic acid or methacrylic acid to produce a compound having the formula $A-S-CH_2-\overset{R_1}{\overset{|}{C}}H-\overset{O}{\overset{\|}{C}}-OH$ wherein $R_1$ is hydrogen or methyl, respectively, with the proviso that when $R_1$ is methyl, step (C) is performed,

(C) resolving the product of step (B) to produce a compound in which $-CH_2-\overset{R_1}{\overset{|}{C}}H-$ is in the D- configuration,

(D) reacting the product of step (B) or (C) with an R coupling compound,

(E) coupling $R_2$ to the product of step (D);

XX. a method comprising the steps of

(A) reacting a compound having the formula R-A-SH with a compound having the formula $X-(CH_2)_n-\overset{R_1}{\overset{|}{C}}H-\overset{O}{\overset{\|}{C}}-OH$, wherein X is bromo, chloro or iodo,

(B) coupling $R_2$ to the product of step (A) to produce a compound having the formula $R-A-S-(CH_2)_n-\overset{R_1}{\overset{|}{C}}H-\overset{O}{\overset{\|}{C}}-R_2$ wherein $R_1$ is hydrogen or methyl, with the proviso that when n is 1 and $R_1$ is methyl, step (C) is performed,

(C) resolving the product of step (B) to produce a compound in which $-(CH_2)_n-\overset{R_1}{\overset{|}{C}}H-$ is in the D- configuration;

XXI. a method comprising the steps of

(A) reacting a compound having the formula $R_3-SH$ with acrylic acid or methacrylic acid to produce a compound having the formula $R_3-S-CH_2-\overset{R_1}{\overset{|}{C}}H-\overset{O}{\overset{\|}{C}}-OH$, wherein $R_1$ is hydrogen or methyl, respectively, with the proviso that when $R_1$ is methyl, step (B) is performed,

(B) resolving the product of step (A) to produce a compound in which $-CH_2-\overset{R_1}{\overset{|}{C}}H-$ is in the D- configuration,

(C) coupling $R_2$ to the product of step (A) or (B),

(D) removing the $R_3$ protecting group from the product of step (C),

(E) reacting the product of step (D) with a compound having the formula R-A;

XXII. a method comprising the steps of

(A) reacting a compound having the formula A-SH with acrylic acid or methacrylic acid,

(B) reacting the product of step (A) with an R coupling compound to produce a compound having the formula

$R-A-S-CH_2-\overset{R_1}{\overset{|}{CH}}-\overset{O}{\overset{\|}{C}}-OH$, wherein $R_1$ is hydrogen or methyl, with the proviso that when $R_1$ is methyl, step (C) is performed,

(C) resolving the product of step (B) to produce a compound in which $-CH_2-\overset{R_1}{\overset{|}{CH}}-$ is in the D- configuration,

(D) coupling $R_2$ to the product of step (B) or (C);

XXIII. a method comprising the steps of

(A) reacting a compound having the formula

$H_3C-\overset{O}{\overset{\|}{C}}-S-(CH_2)_n-\overset{R_1}{\overset{|}{CH}}-\overset{O}{\overset{\|}{C}}-OH$ with an o-nitrobenzyl alcohol to produce a compound having the formula

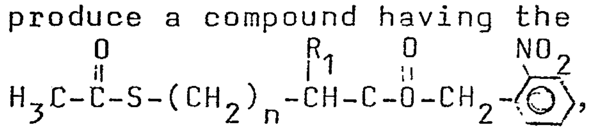, wherein $R_1$ is hydrogen or methyl, with the proviso that when n is 1 and $R_1$ is methyl, step (B) is performed,

(B) resolving the product of step (A) to produce a compound in which $-(CH_2)_n-\overset{R_1}{\overset{|}{CH}}-$ is in the D- configuration,

(C) removing the o-nitrobenzyl group from the product of step (A) or (B) by irradiation,

(D) coupling $R_2$ to the product of step (C),

(E) removing the acetyl group from the product of step (D),

(F) reacting the product of step (E) with a compound having the formula R-A;

XXIV. a method comprising the steps of

(A) reacting a compound having the formula

$H_2N-(CH_2)_n-\overset{R_1}{CH}-\overset{O}{C}-OH$, wherein $R_1$ is hydrogen or methyl, with H-X, wherein X is bromine or chlorine to produce a compound having the formula $X-(CH_2)_n-\overset{R_1}{CH}-\overset{O}{C}-OH$, with the proviso that when n is 1 and $R_1$ is methyl, the starting compound is resolved to produce a compound in which $-(CH_2)_n-\overset{R_1}{CH}-$ is in the D- configuration prior to reaction with H-X,

(B) reacting the product of step (A) with a compound having the formula R-A-SH,

(C) coupling $R_2$ to the product of step (B);

XXV. a method comprising the steps of

(A) reacting a compound having the formula $H_2N-(CH_2)_n-\overset{R_1}{CH}-\overset{O}{C}-OH$, wherein $R_1$ is hydrogen or methyl, with H-X, wherein X is bromine or chlorine, to produce a compound having the formula $X-(CH_2)_n-\overset{R_1}{CH}-\overset{O}{C}-OH$, with the proviso that when n is 1 and $R_1$ is methyl, the starting compound is resolved to produce a compound in which $-(CH_2)_n-\overset{R_1}{CH}-$ is in the D- configuration prior to reaction with H-X,

(B) reacting the product of step (A) with thiobenzoic acid or thioacetic acid,

(C) removing the benzoyl or acetyl group from the product of step (B),

(D) reacting the product of step (C) with a compound having the formula R-A,

(E) coupling $R_2$ to the product of step (D).

XXVI. a method comprising the steps of

(A) reacting a compound having the formula $HO-(CH_2)_n-\overset{R_1}{CH}-\overset{O}{C}-OH$, wherein $R_1$ is hydrogen or methyl, with $PBr_3$, to produce a compound having the formula $Br-(CH_2)_n-\overset{R_1}{CH}-\overset{O}{C}-OH$, with the proviso that when n is 1 and $R_1$ is methyl, the starting compound is resolved to produce a compound in which $-(CH_2)_n-\overset{R_1}{CH}-$ is in the D- configuration

prior to reaction with $PBr_3$,

(B) reacting the product of step (A) with a compound having the formula R-A-SH,

(C) coupling $R_2$ to the product of step (B).

XXVII a method comprising the steps of

(A) reacting 3-benzoylthio-2-D-methylpropionic acid with 2-methylpropene to produce 3-benzoylthio-2-D-methyl-propionic acid t-butyl ester,

(B) removing the benzoyl group from the product of step (A),

(C) reacting the product of step (B) with a compound R(or R')-A to produce a compound having the formula 3-[R(or R')-A]thio-2-D-methylpropionic acid t-butyl ester,

(D) removing the t-butyl ester group from the product of step (C),

(E) coupling the product of step (D) with $R_2$.

XXVIII a method comprising the steps of

(A) reacting 3-mercapto-2-D-methylpropionic acid t-butyl ester with a compound having the formula $R_3$-A to produce a compound having the formula 3-($R_3$-A)thio-2-D-methylpropionic acid t-butyl ester,

(B) removing the $R_3$ protecting group from the product of step (A),

(C) reacting the product of step (B) with an R or R' coupling compound to produce a compound having the formula 3-[R(or r')-A]thio-2-D-methylpropionic acid t-butyl ester,

(D) removing the t-butyl ester group from the product of step (C),

(E) coupling the product of step (D) with $R_2$;

XXIX. a method comprising the steps of

(A) removing the benzoyl group from 3-benzoylthio-2-D-methylpropionic acid,

(B) reacting the product of step (A) with a compound having the formula $R_3$-A to produce a compound having the formula 3-($R_3$-A)thio-2-D-methylpropionic acid,

(C) removing the $R_3$ protecting group from the product of step (B),

(D) reacting the product of step (C) with an R or R'

coupling compound to produce a compound having the formula 3-[R(or R')-A]thio-2-D-methylpropionic acid,

(E) coupling the product of step (D) with $R_2$;

XXX. a method comprising the steps of

(A) reacting 3-mercapto-2-D-methylpropionic acid with a compound having the formula $R_3$-A to produce a compound having the formula 3-($R_3$-A)thio-2-D-methylpropionic acid,

(B) coupling the product of step (A) with $R_2$,

(C) removing the $R_3$ protecting group from the product of step (B),

(D) reacting the product of step (C) with an R or R' coupling compound; and

XXXI. a method comprising the steps

(A) coupling a compound having the formula 3-($R_2$-A) thio-2-D-methylpropionic acid with $R_2$,

(B) removing the $R_3$ protecting group from the product of step (A),

(C) reacting the product of step (B) with an R or R' coupling compound.

2. The method of claim 1 wherein R is benzoyl, A is phenylalanyl, $R_1$ is methyl, $R_2$ is L-proline and n is 1.

3. The method of claim 1 wherein method IV is selected and step (A) includes the proviso that when R is R' and the amino acid is in the D- or L- form in the compound prepared by this process, R is $R_3$ in compound R-A, and the $R_3$ protecting group is removed from R-A-SH and the resulting A-SH is reacted with an R' coupling compound prior to step (B).

4. The method of claim 1 wherein method VII is selected and step (B) includes the proviso that when R is R' and the amino acid is in the D- or L- form in the compound prepared by this process, R is $R_3$ in compound R-A, and the $R_3$ protecting group is removed from the compound having the formula R-A-S-$CH_2$-$\overset{R_1}{\overset{|}{C}}$H-$\overset{O}{\overset{\|}{C}}$-OH, and prior to step (E) the product of step (D) is reacted with an R' coupling compound.

5. A compound having the first formula given in claim 1 when prepared by the method of any of claims 1 - 4.

6. The compound of claim 5 for use as an inhibitor of angiotensin converting enzyme.

0038117

European Patent Office

# EUROPEAN SEARCH REPORT

Application number
EP 81 30 0341

## DOCUMENTS CONSIDERED TO BE RELEVANT

| Category | Citation of document with indication, where appropriate, of relevant passages | Relevant to claim |
|---|---|---|
| | GB - A - 2 050 359 (E.R. SQUIBB and SONS)<br>* Whole document *<br>-- | 1-6 |
| A | FR - A - 2 407 204 (SANDOZ)<br>* Claims *<br>-- | 1 |
| PX | EP - A - 0 009 898 (UNIVERSITY OF MIAMI)<br>* Whole document *<br>---- | 1-6 |

CLASSIFICATION OF THE APPLICATION (Int. Cl.³)

C 07 D 207/16
C 07 C 103/52
C 07 C 153/07
A 61 K 37/64
C 07 D 277/06

TECHNICAL FIELDS SEARCHED (Int. Cl.³)

C 07 D 207/16
277/06
A 61 K 37/64

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 12-06-1981 | BRIGHENTI |

EPO Form 1503.1 06.78